# EUROPEAN PATENT SPECIFICATION

(11) **EP 1 123 301 B1**
(45) Date of publication and mention of the grant of the patent: **03.12.2003**
(21) Application number: 99953923.2
(22) Date of filing: 22.10.1999
(51) Int. Cl.: C07F 9/38, C07C 229/16, A61K 51/10, A61K 51/08

(54) **CHELATING AGENTS FOR RADIOIMMUNOTHERAPY**
CHELATIERMITTEL FÜR RADIOIMMUNOTHERAPIE
AGENTS CHELATANTS POUR RADIOIMMUNOTHERAPIE

(30) Priority: 23.10.1998 EP 98402648
(43) Date of publication of application: 16.08.2001
(73) Proprietor: INSTITUT NATIONAL DE LA SANTE ET DE LA RECHERCHE MEDICALE (INSERM), 75654 Paris Cédex 13 (FR)
(72) Inventor: GESTIN, Jean-François, F-44470 Mauves-sur-Loire (FR); LOUSSOUARN, Anthony, F-44000 Nantes (FR); FAIVRE-CHAUVET, Alain, F-44300 Reze (FR)
(74) Representative: Grosset-Fournier, Chantal Catherine
(86) International application number: EP9908031
(87) International publication number: WO00024751

(56) References cited:
- US-A- 5 089 663
- US-A- 5 292 938
- LOUSSOUARN A ET AL: "Synthesis of new bifunctional chelating agents: (1R*,2R*,4S*)-4-isothiocyanatocyclohexane- 1,2-diamine-N,N,N',N'-tetra kismethanephosphonic acid (4-ICMP) and (1R*,2R*,4S*)-4-isothiocyanato cyclohexane-1,2-diamine-N,N,N',N'-tetrakis ethanephosphonic acid (4-ICEP)" J. CHEM. SOC., PERKIN TRANS. 1 (JCPRB4,0300922X);1998; (2); PP.237-242, - 21 January 1998 (1998-01-21) XP002097516 Institut de Biologie;INSERM U.463 (ex-U.211); Nantes; 44035; Fr. (FR) cited in the application
- BRECHBIEL M W ET AL: "Preparation of the Novel Chelating Agent N-(2-Aminoethyl)-trans-1,2 -diaminocyclohexane-N,N',N''-pentaacetic Acid (H5CyDTPA), a Preorganized Analog of Diethylenetriaminepentaacetic Acid (H5DTPA), and the Structures of BiIII(CyDTPA)2- and BiIII(H2DTPA) Complexes" INORG. CHEM. (INOCAJ,00201669); VOL.35 (21); PP.6343-6348, - 9 October 1996 (1996-10-09) XP002097517 National Institutes of Health;Chemistry Section; Bethesda; 20892; MD; USA (US)
- LOUSSOUARN A ET AL: "Simple and general procedure for the synthesis of semi-rigid chelating agents for radiometal complexation studies and its application to semi-rigid functionalised ligands (BCA) synthesis" MATER. SCI. FORUM (MSFOEP,02555476);1999; VOL.315-317 (RARE EARTHS '98); PP.262-267, XP000865647 Institut Biologie;Nantes; F-44035; Fr. (FR)

## Description

The invention relates to compounds useful as chelating agents, complexes between said compounds and radioelements, and to their uses, in particular in pharmaceutical compositions and compositions for the diagnosis of pathologies such as cancers.

Immunotherapy with radiolabeled antibodies should allow fairly specific targeting of certain cancers (Schubiger et al., 1996; Parker, 1990). However, iodine-131 (Bardies et al., 1992; Stein et al., 1995) may not be the best isotope for tumor therapy because of its limited specific activity, low beta-energy, relatively long half-life and strong gamma emission.

Another approach to improving therapeutic efficacy is the use of replacement isotopes with better physical properties. Chelators that can hold radiometals with high stability under physiological conditions are essential to avoid excessive radiation damage to non-target cells. Moreover, the development of new bifunctional chelating agents is essential for this purpose. Thus synthesis of new chelating agents able to bind radiometals such as rhenium-188, yttrium-90, samarium-153 or Bismuth-213 and in general all the α and β particles emitters will be required to possess sufficiently stable chelators.

Accordingly, one of the aims of the invention is to provide chelating agents forming stable complexes *in vivo* with the numerous potential candidates for such applications.

The stability of a non-macrocyclic ligand can be favourably influenced by the preorganization of the open chain. In fact, a semi-rigid structure such as that of *trans* 1-2 diaminocyclohexane limits the rotation of the ethylene bridge, so that the purpose of the cyclohexane design is to preorient the four pendent arms in a skew position.

A first investigation (Mease et al., 1990), which was guided by a study performed on polyaminocarboxylic acid ligands incorporating the skeleton of ethylenediaminetetraacetic acid (EDTA) in a cyclohexane structure, showed the influence of this semi-rigid structure on the stability of the resulting complexes. A second study (Goeckeler et al., 1987) of the stability of lanthanides as ¹⁵³Sm-polyaminophosphonic acid complexes showed that ethylenediamine tetramethylphosphonic acid (EDTMP) derivatives allow stable quantitative ¹⁵³Sm chelation.

The (1*R**, 2*R**, 4*S**)-4-acetamido-1,2-diaminocyclohexane dihydro chloride compound, the structural derivative of *trans*-1,2-diaminocyclohexane, have been prepared (Gestin et al., 1997; Loussouarn, et al., 1998). This intermediate, which is functionalized at position 4 of the cycle by a protected amine termination (Meares et al. , 1984) for future covalent attachment to biomolecules, allows the introduction of different chelating groups via the free amines.

US 5 292 938 concerns the preparation of forms of CDTPA and in particular CDTA wherein R is NH₂, -N=C=S, -NH(CO)CH₂X.

Brechbiel H. W. et al reports the preparation of H₅C_{y}DTPA and the synthesis and structural characterisation of Bismuth (III) complexes of C_{y}DTPA⁵⁻.

US 5 089 663 concentrates on the preparation of derivatives of the semi rigid chelate cyclohexyl EDTA (CDTA).

The Inventors have developed a new simple and efficient synthesis pathway from *trans* -1,2- diaminocyclohexane to provide access to a new class of semi-rigid chelating agents. This same reactional scheme applies to the reactional intermediary, (1*R**, 2*R**, 4*S**)-4-acetamido-1,2-diaminocyclohexane dihydrochloride, which allows the synthesis of these same chelating agents, though functionalized back of the cycle by a termination allowed coupling to an antibody or any other biological substance such as a hapten.

The present invention relates to compounds of the following formula (I) : 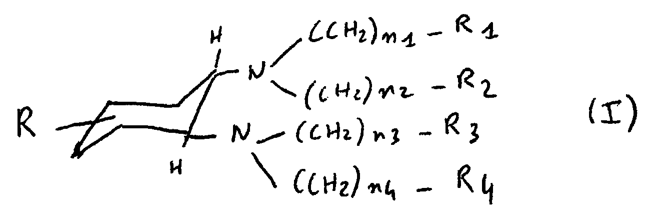 in which :
- n₁, n₂, n₃ and n₄, independently from each other, represent an integer from 1 to 5, preferably from 1 to 3,
- R₁, R₂, R₃ and R₄, independently from each other, represent :
   . -COOH,
   . -PO(OH)₂, 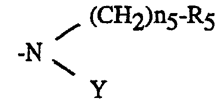
wherein n₅ represents an integer from 1 to 5, preferably from 1 to 3, R₅ represents -COOH or -PO(OH)₂, and Y represents H or a group -(CH2)n₆-R₆ in which n₆ represents an integer from 1 to 5, preferably from 1 to 3, and R₆ represents -COOH or -PO(OH)₂,
provided that at least one of R₁, R₂, R₃ or R₄ represents a group 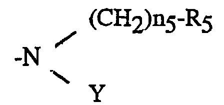 such as defined above,
- R represents :
   . H, or -NHCOCH₃, or
   . a group carrying a function liable to bind, if necessary via a binding site, to molecules, such as antibodies, haptens or peptides, which are able to bind specifically with epitopes located at the surface of the cells of the organism, or to chemical or biological compounds located at the surface of a solid carrier, or
   . a group carrying a function linked, if necessary via a binding site, to molecules as defined above,
   the two following compounds, CDTPA and CTTHA, being excluded :

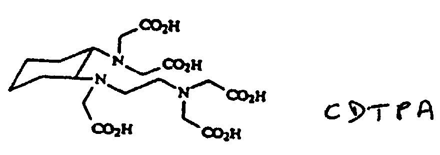

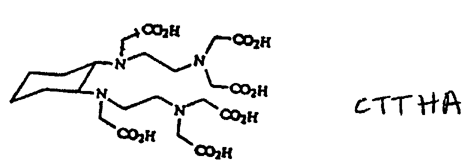

The invention relates more particularly to compounds of formula (I) such as defined above, characterized in that :
- when R₁, R₂, R₃ or R₄ represents -COOH or -PO(OH)₂, then n₁, n₂, n₃ or n₄ represents 1 respectively,
- when R₁, R₂, R₃ or R₄ represents a group 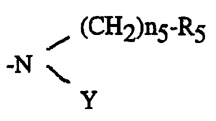 then n₁, n₂, n₃ or n₄ represents 2 or 3 respectively, and preferably 2,
- n₅, and optionally n₆, represents 1.

The invention also relates more particularly to compounds of formula (I) such as defined above, characterized in that at least one, and more preferably two of R₁, R₂, R₃ and R₄, represent a group 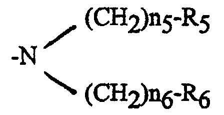 wherein n₅, n₆, R₅ and R₆ are defined above.

Preferred compounds of formula (I) such as defined above, wherein R is different from hydrogen, are compounds of the following formula (II) : 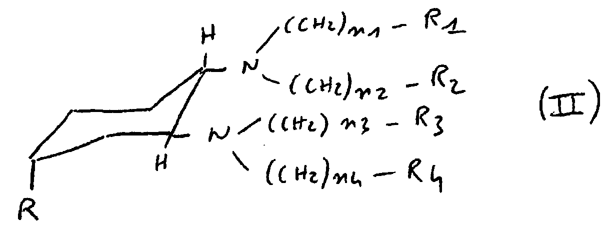 wherein n₁, n₂, n₃, n₄, R₁, R₂, R₃, R₄ and R are such as defined above.

The invention relates more particularly to compounds of formula (I) or (II) such as defined above, characterized in that R represents a group carrying a function liable to bind, if necessary via a binding site, to molecules, such as antibodies, haptens or peptides, as defined above, and in particular R represents a group chosen among all the coupling functions for vector or solid support binding, such as the following groups :
. alcohol group, such as -OH,
. amino group, such as -NH₂, -NO₂,
. aldehyde group, such as -CHO,
. carboxylic group, such as -COOH,
. anhydride group, such as -CO-O-CO-R",
. -CO-CH₂X, wherein X represents an halogen atom, such as Cl or Br,
. -CO-X, wherein X represents an halogen atom, such as Cl or Br,
. a diazonium ion N₂⁺,
. an activated ester, such as -COOR", R" = ethyl or N-hydrosuccinimide,
. a sulfonic group, such as SO₃H,
. a thiocyanate group, such as -NCS, or an isocyanate -NCO, or a -NH-NCS group
. a thiol group, such as -SH,
. a disulfure group, such as -S-S-R".

The invention also concerns compounds of formula (I) or (II) such as defined above, characterized in that R represents a group carrying a function linked, if necessary via a binding site, to molecules, such as antibodies, haptens or peptides, as defined above, and more particularly R represents a group chosen among the following groups :
. -O-CO-R',
. -NH-CO-R',
. -NH-CS-R',
. -CH=N-R',
. -CO-NH-R',
. -CO-CH₂-NH-R',
. -N = N-NH-R',
. -SO₂-NH-R',
. -NH-CS-NH-R',
. -thioether-R',
. -CO-S-R',
. -CO-CH₂-S-R',
. -S-S-R',
. -NH-CH₂-R',
. -CO-NH-N=CH-R',
. -CS-NH-N=CH-R',
wherein R' represents said molecule.

The invention concerns more specifically compounds such as described above of the following formula (III) : 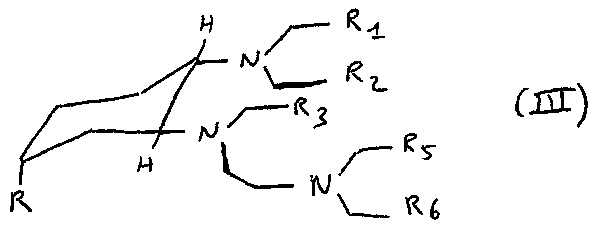 in which R₁, R₂, R₃, R₅ and R₆ independently from each other represent - COOH or -PO(OH)₂, and R is a group as defined above.

Preferred compounds of formula (III) are such that :
. R₁ = R₅ = R₆ = COOH and R₂, = R₃ = PO(HO)₂, or
. R₁ = R₂ = R₃ = R₅ = R₆ = COOH, or
. R₁ = R₂ = R₃ = R₅ = R6 = PO(OH)₂.

The invention also concerns more specifically compounds such as described above, of the following formula (IV) : 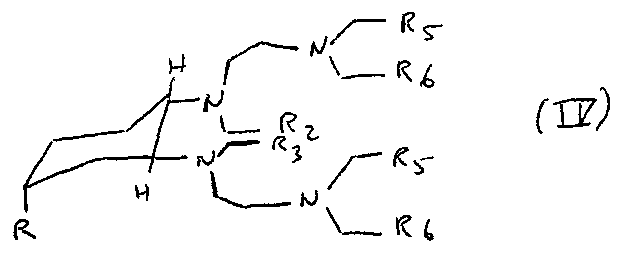 wherein R₂, R₅ and R₆, independently from each other, represent -COOH or -PO(OH)₂, and R is a group as defined above.

Preferred compounds of formula (IV) are such that :
. R₂ = R₃ = PO(OH)₂, and R₅ = R₆ = COOH, or
. R₂ = R₃ = R₅ = R₆ = COOH, or
. R₂ = R₃ = R₅ = R₆ = PO(OH)₂.

The invention also relates to complexes between a compound such as described above, and a radioactive element, said complexes resulting from the association of said radioelement with the -COOH and/or -PO(OH)₂ groups of said compound (the bonds between said radioelement and said compound being ionic bonds).

The above-mentioned radioelements are more particularly α, β or γ emitter radiometals, and preferably from the groups of actinides or lanthanides.

The invention relates more particularly to complexes such as described above, characterized in that said radioelements are α or β emitter radiometals (susceptible to be used in therapy, and more particularly in radioimmunotherapy in the frame of cancer treatments).

Advantageously, α emitter radiometals are chosen among the following : Actinium 225, Bismuth 213.

Advantageously, β emitter radiometals are chosen among the followings : ³³P, ¹⁹⁹Au, ¹²¹Sn, ¹⁷⁷Lu, ⁶⁷Cu, ¹⁰⁵Rh, ⁴⁷Sc, ⁷⁷As, ¹⁵³Sm, ¹⁵⁹Gd, ¹⁴³Pr, ¹⁸⁶Re, ¹¹¹Ag, ¹⁴⁹Pm, ¹⁰⁹Pd, ¹⁶⁶Ho, ³²P, ¹⁸⁸Re, ¹⁹⁴Ir, ¹⁴²Pr, ⁹⁰Y.

Preferred complexes with radiometals used in therapy, as defined above, are such that the compound is chosen among those wherein R represents a group carrying a function linked, if necessary via a binding site, to molecules as defined above, and more particularly among those compounds wherein the group R comprises :
- an antibody (polyclonal or monoclonal) liable to recognize and to bind to specific epitopes on the surface of specific cells of the organism,
- or an hapten, i.e. a non-immunogenic molecule of low MW capable of inducing the production of antibodies against itself, said hapten being liable to recognize and to bind to one or several molecules already bound (in a first step of the treatment) to epitopes on the surface of specific cells in the organism,
- or a peptide resulting from the association of different amino acids and liable to recognize and to bind to specific epitopes on the surface of specific cells of the organism.

The invention also concerns the use of a complex such as described above, for the manufacture of a medicament for radioimmunotherapy (also called radiopharmaceutical), in particular for the treatment of cancers, or for the treatment against metastase proliferation.

More particularly, the invention relates to the use of a complex such as defined above, for the manufacture of a medicament for the treatment of :
- lung cancer, said complex preferably being such that it comprises a radioelement chosen among : ¹⁸⁸Re, ¹⁸⁶Re, ¹⁵³Sm, ⁶⁷Cu and ⁹⁰Y, and
wherein R comprises an antibody specific for lung cancer cells, such as Anti N-CAM Antibody, Anti CEA Antibody, Anti Carbohydrates Antibodies, or an hapten chosen among Anti N-CAM-679 Bispecific antibody, Anti CEA-679 Bispecific antibody, Anti Carbohydrates-679 Bispecific antibody, Anti N-CAM-734 Bispecific antibody, Anti CEA-734 Bispecific antibody, Anti Carbohydrates-734 Bispecific antibody,
- liver and pancreatic cancers, said complex preferably being such that it comprises a radioelement chosen among those cited above in the case of lung cancer, and wherein R comprises an antibody specific for liver and pancreatic cancer cells, such as antibodies and haptens described above in the case of lung cancer,
- ovarian cancer, said complex preferably being such that it comprises a radioelement chosen among those cited above in the case of lung cancer, and
wherein R comprises an antibody specific for ovarian cancer cells, such as OC125, MOV18, MOV19, OVTL3, or an hapten chosen among OC125-679 Bispecific antibody, MOV18-679 Bispecific antibody, MOV19-679 Bispecific antibody, OVTL3-679 Bispecific antibody, OC125-734 Bispecific antibody, MOV18-734 Bispecific antibody, MOV19-734 Bispecific antibody, OVTL3-734 Bispecific antibody,
- bladder cancer, said complex preferably being such that it comprises a radioelement chosen among those cited above in the case of lung cancer, and
wherein R comprises an antibody specific for bladder cancer cells, such as AC48-127, or an hapten chosen among 48-127 Bispecific antibody, 48-127-679 Bispecific antibody, 48-127-734 Bispecific antibody,
- colorectal cancer, said complex preferably being such that it comprises a radioelement chosen among those cited above in the case of lung cancer, and
wherein R comprises an antibody specific for colorectal cancer cells, such as Anti CEA Antibody, Anti Carbohydrates Antibodies, or an hapten chosen among Anti CEA-679 Bispecific antibody, Anti Carbohydrates-679 Bispecific antibody, Anti CEA-734 Bispecific antibody, Anti Carbohydrates-734 Bispecific antibody,
- thyroid medullary cancer, said complex preferably being such that it comprises a radioelement chosen among those cited above in the case of lung cancer, and wherein R comprises an antibody specific for thyroid medullary cancer cells, such as Anti CEA Antibody, or an hapten chosen among Anti CEA-679 Bispecific antibody, Anti CEA-734 Bispecific antibody,
- lymphoma, said complex preferably being such that it comprises a radioelement chosen among : ²¹³Bi, ²²⁵Ac, ¹⁵³Sm, and ⁶⁷Cu, and wherein R comprises an antibody specific for lymphoma cells, such as specific antibody against expressed antigens surfaces lymphocyte cells, e.g. CD19, CD37, or an hapten such as bispecific antibody against expressed antigens surfaces lymphocyte cells, e.g. CD19-679, CD37-679, CD19-734, CD37-734,
- myeloma, said complex preferably being such that it comprises a radioelement chosen among : ²¹³Bi, ²²⁵Ac, ¹⁵³Sm, and ⁶⁷Cu, and wherein R comprises an antibody specific for myeloma cells, such as specific antibody against expressed antigens surfaces myeloma cells, e.g. BB4, or an hapten such as bispecific antibody against expressed antigens surfaces myeloma cells, BB4-679, BB4-734,
- osteoarticular pathology, particularly in bone cancer extension balance.

The invention also concerns pharmaceutical compositions characterized in that they comprise an effective amount of a complex such as described above, in association with a suitable pharmaceutical carrier.

Pharmaceutical compositions according to the invention are more particularly characterized in that they are in a form suitable for an IV or IP administration in located areas.

Preferred pharmaceutical compositions according to the invention, are characterized in that the daily dosage is comprised between 1 and 100MBq /kg, e.g. between 3,7 and 74MBq/kg.

The invention also relates to complexes, as defined above, between a compound such as described above, and a radioactive element, characterized in that the radioelements are γ emitter radiometals (i.e. radiometals susceptible to be used in diagnosis methods, such as radioimmunoscintigraphy).

Advantageously, said radiometals are chosen among ¹¹¹In, ^{99m}Tc, ⁶⁴Cu.

Preferred complexes with radiometals used in diagnosis, as defined above, are such that the compound is chosen among those wherein R represents a group carrying a function linked, if necessary via a binding site, to molecules as defined above, and more particularly among those compounds wherein the group R comprises :
- an antibody (polyclonal or monoclonal) liable to recognize and to bind to specific epitopes on the surface of specific cells of the organism,
- or an hapten, i.e. a non-immunogenic molecule of low MW capable of inducing the production of antibodies against itself, said hapten being liable to recognize and to bind to one or several molecules already bound (in a first step of the method of diagnosis) to epitopes on the surface of specific cells in the organism,
- or a peptide resulting from the association of different amino acids and liable to recognize and to bind to specific epitopes on the surface of specific cells of the organism.

The invention also concerns the use of a complex such as described above, for carrying out diagnosis methods such as radioimmunoscintigraphy.

More particularly, the invention concerns the use of a complex such as defined above, for carrying out the following diagnosis methods by radioimmunoscintigraphy :
- diagnosis of cancers, such as cited above, the complex used being preferably such that it comprises ¹¹¹In, or 99mTc as radioelements, and R comprises an antibody or a hapten specific for such cancer cells, such as antibodies or haptens mentioned above in the frame of the cancers listed above,
- diagnosis of cardiovascular diseases, such as graft rejection, myocardic infarcts,
- diagnosis of cerebral diseases,
- diagnosis of renal diseases, in particular in the study of individual kidney functions, location of ectopic kidney, renal filtration and secretion troubles,
- vascular diseases, such as embolism and thrombosis, the complex used being preferably such that it comprises ¹¹¹In, or ^{99m}Tc as radioelements, and R comprises an antibody such as anti platelets or anti fibrin antibodies.

The invention also concerns the use of the complexes defined above for carrying out bone scintigraphy, in particular in the frame of the diagnosis of osteoarticular pathology, particularly in bone cancer extension balance.

The invention also relates to methods for the *in vitro* diagnosis of pathologies listed above, characterized in that it comprises the steps of incubating a biological sample, such as serum, plasma urines, with a complex as described above, the components of the biological sample being fixed to a solid carrier, rinsing the solid carrier and detecting the γ emission of the complex bound to the components of the sample on the solid carrier.

The invention also concerns the kits for carrying out said diagnosis methods, said kits comprising complexes as described above according to the invention.

The invention also relates to the use of a compound of formula (I) defined above, included compounds CDTPA and CTTHA, for the manufacture of a medicament for the treatment of pathologies where ionic imbalances occur, or against the formation of stones in the organism.

The invention also relates to the use of a compound of formula (I) defined above, included compounds CDTPA and CTTHA, for carrying out a process for the detoxication of polluted medium, such as liquid phases polluted by bivalent or trivalent metals radioactives or not.

The invention also concerns a process for the detoxication of a polluted medium comprising the steps of contacting said medium with a compound as defined above, advantageously itself bound to a solid carrier, and recovering said medium substantially free of contaminants which are bound to said compound on the solid carrier.

The invention also relates to the use of a compound of formula (I) defined above, included compounds CDTPA and CTTHA, for carrying out a process for the radionuclides purification, said compound of the invention being bound to a solid phase.

The invention also relates to the use of a complex between of formula (I) defined above, included compounds CDTPA and CTTHA, for carrying out a bone scintigraphy, in particular in the frame of the diagnosis of osteoarticular pathology, particularly in bone cancer extension balance.

The invention also relates to processes for preparing compounds and complexes as described above.

A process for the preparation of compounds according to the invention, comprises the following steps :

The invention also relates to processes for preparing compounds and complexes as described above. A process for the preparation of compounds according to the invention, comprises the following steps :
- contacting trans-1,2-diaminocyclohexane of the following formula a : 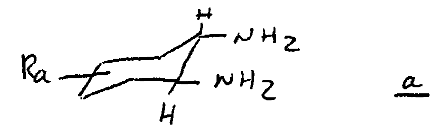 wherein Rₐ is H or NHCOCH₃,
   * either with vinyl propionate, preferably by stirring 20h at room temperature, leading to the following compound b 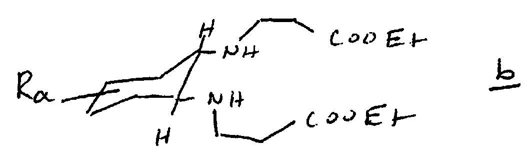
      . contacting compound b with X-(CH₂)ₙ-COOEt, wherein X represents an halogen atom, and n represents an integer from 1 to 5, preferably at reflux during 15h, leading to the following compound c 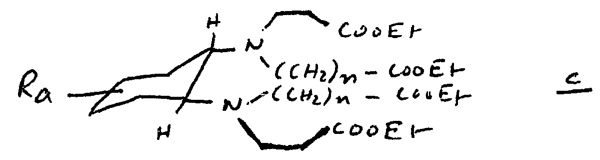
      . treating compound c with HCl, preferably 6N HCl at reflux overnight, leading to the following compound d 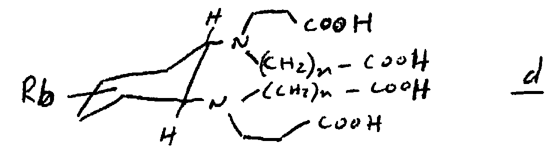 wherein R_{b} represents H or NH₂,
   * or with diethyl vinyl phosphonate, preferably by stirring 15h at reflux, leading to the following compound e 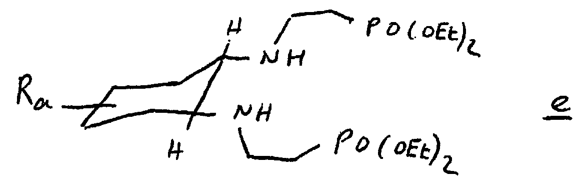
      . contacting compound e with X-(CH₂)ₙ-COOEt, wherein X represents an halogen atom, and n represents an integer from 1 to 5, preferably at reflux during 15h, leading to the following compound f 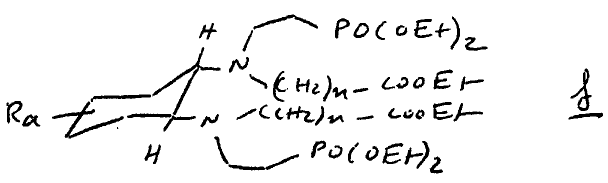
      . treating compound f with HCl, preferably 6N HCl at reflux overnight, leading to the following compound g 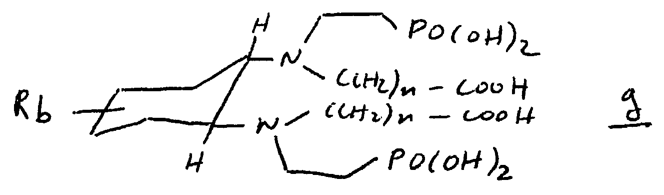 wherein R_{b} represents H or NH₂,
      . if desired, treating compound g with phosphorous acid, preferably by stirring 30 mn at 80°C, leading to the following compound h

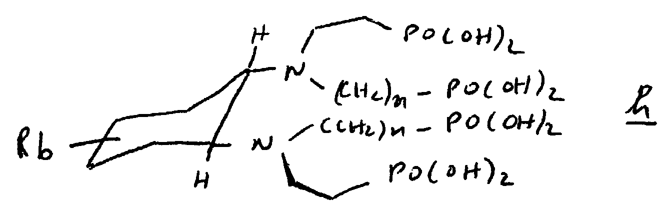

Another process for the preparation of compounds according to the invention, comprises the following steps :
- contacting the compound of formula a described above with a compound of formula H₂C=CH-(CH₂)_{nA}-CN wherein nA = 0 (acrylonitrile), or nA represents a integer from 1 to 3, preferably at room temperature during 20h, leading to the following compound i 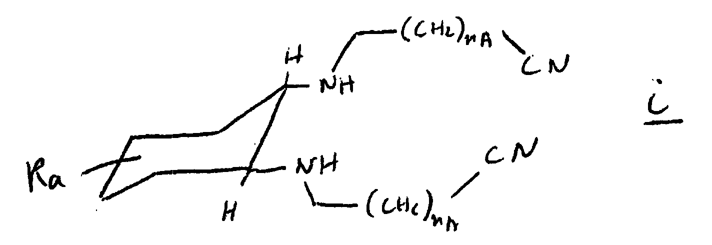
- contacting compound i with the following compound i 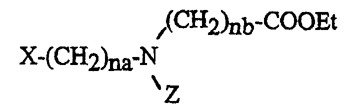 wherein X represents an halogen atom, na and nb, independently from each other represent an integer from 1 to 5, Z represents H or (CH₂)_{nc}-COOEt, and nc represents an integer from 1 to 5, preferably at 70°C during 2 days, leading to the following compound k 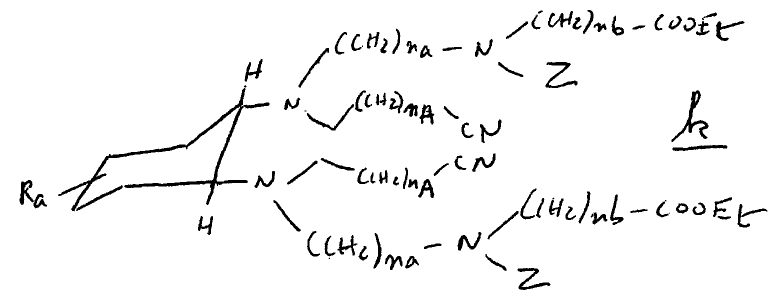
   . treating compound k with HCl, preferably 6N HCl at reflux overnight, leading to the following compound 1 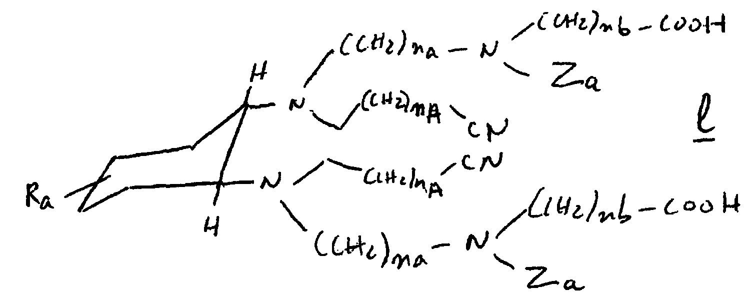 wherein Za represents H or -(CH2)nb-COOH, nA, na and nb being such as defined above, and R_{b} represents H or NH₂.

Another process for the preparation of compounds according to the invention, comprises the following steps :
- contacting the compound of formula a described above with paraformaldehyde and diethylphosphite, preferably in THF at reflux during 4h, leading to the following compound m 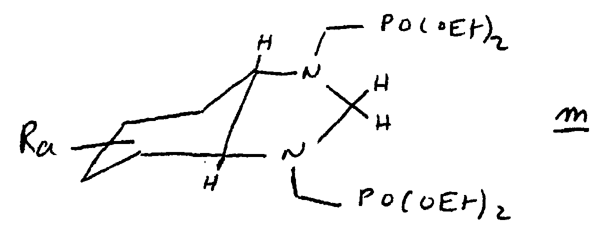
- treating compound m with HCl, preferably 3N HCl in MeOH at 50°C overnight, leading to the following compound n 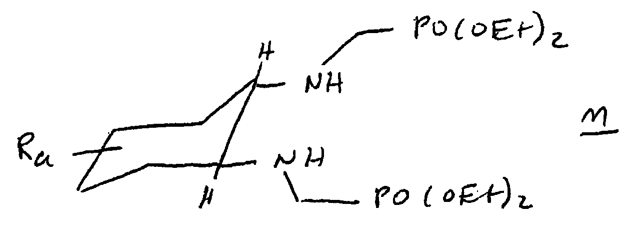
- contacting compound n :
   * either with 1 equivalent of compound i 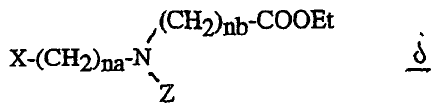 as described above, preferably at 70°C during 2 days, leading to the following compound of formula o 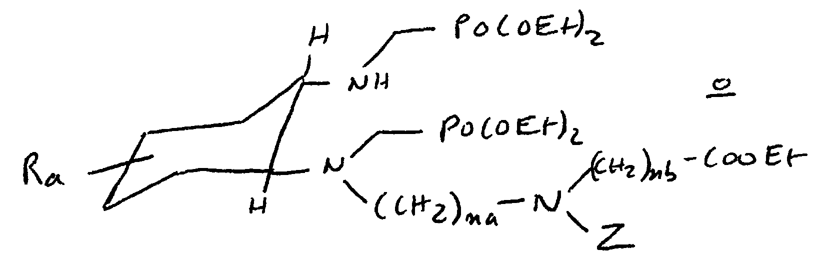
      . contacting compound o with X-(CH₂)ₙ-COOEt, wherein X represents an halogen atom, and n represents an integer from 1 to 5, preferably at reflux during 15h, leading to the following compound p 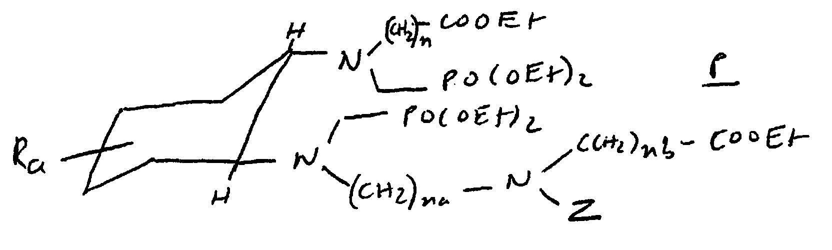
      . treating compound p with HCl, preferably 6N HCl at reflux overnight, leading to the following compound q 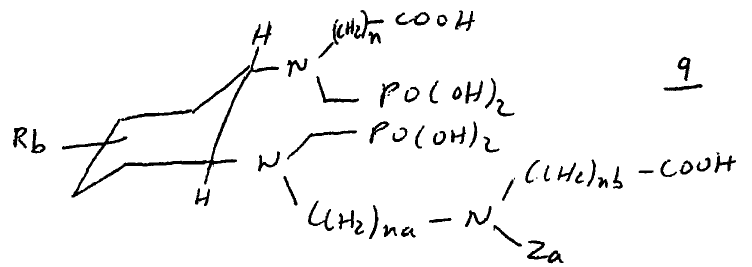 wherein Za represents H or -(CH₂)_{nb}-COOH, na, nb and n being such as defined above, R_{b} represents H or NH₂,
      . if desired, treating compound q with phosphorous acid, preferably by stirring 30 mn at 80°C, leading to the following compound r 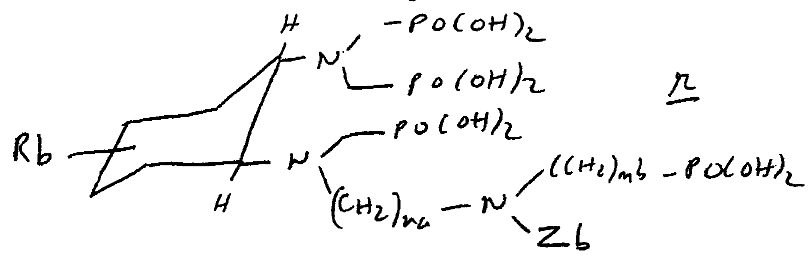
      wherein Zb represents H or -(CH₂)_{nb}-PO(OH)₂
   * or with 2 equivalents of compound j 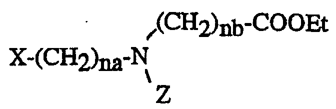 as described above, preferably at 70°C during 2 days, leading to the following compound of formula s 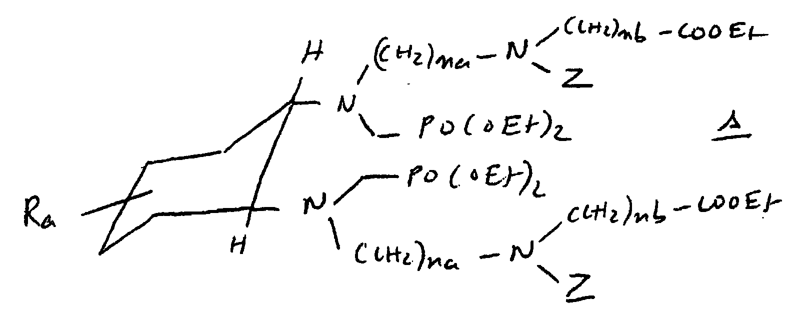
      . treating compound s with HCl, preferably 6N HCl at reflux overnight, leading to the following compound t 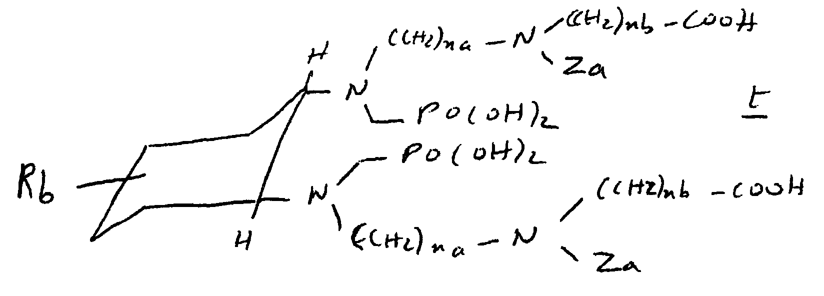 wherein Za represents H or -(CH₂)_{nb}-COOH, na, nb and n being such as defined above, R_{b} represents H or NH₂,
      . if desired, treating compound t with phosphorous acid, preferably by stirring 30 mn at 80°C, leading to the following compound u 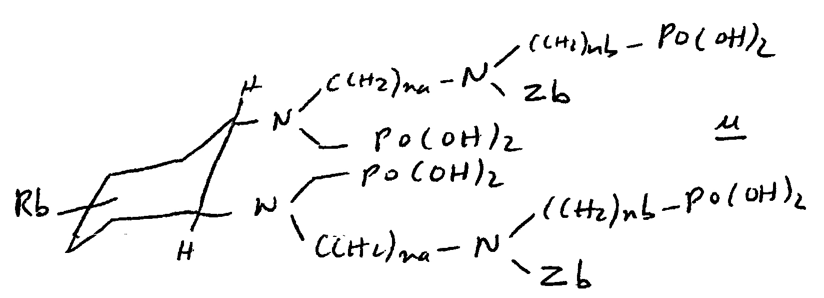 wherein Zb represents H or -(CH₂)_{nb}-PO(OH)₂.

Compound of formula a can be obtained according to the method described in Gestin et al., 1997, and Loussouam et al., 1998.

Compounds wherein Rb represents NH₂ obtained according to the processes described above, can then be transformed in order to correspond to compounds of formula (I) wherein R represents a group carrying a function liable to bind, if necessary via a binding site, to molecules as defined above.

By way of example, compounds of formula (I) wherein R represents - N=C=S, can be obtained by treatment of said compounds wherein Rb represents NH₂ with CSCl2, preferably in acidic or basic conditions.

Compounds of formula (I) wherein R represents a group carrying a function linked, if necessary via a binding site, to molecules as defined above, can then be obtained by coupling said compounds, wherein R represents a group carrying a function liable to bind to said molecules, with said molecules.

Complexes according to the invention are advantageously obtained by incubating the compounds with the radioelements at 37°C during 3 hours.

The invention will be further illustrated in the following examples for the preparation of compounds and complexes according to the invention.

In order to save the functionalized reactional intermediate, i.e. the (1*R**, 2*R**, 4*S**)-4-acetamido-1,2-diaminocyclohexane dihydro chloride compound, the commercial product, *trans*-1,2- diaminocyclohexane **1**, has been used as starting material.

Two approach routes to differently substituted amines were carried out successfully.
- The first depicted in schemes I and II was the Michael type addition of primary amines to some vinylic derivatives to provide monoaddition with high selectivity (Bergeron et al., 1981), allowing N-alkylation to be envisaged at this step. In strategy depicted in scheme **I**, compounds **2a** and **2b** were alkylated by ethyl bromoacetate under conditions recommended by Studer (Studer and Meares, 1992) (KI and Na₂CO₃) to give tetraesters **3a** and **3b**. Acid-catalysed hydrolysis of the ester functions was performed in 3M hydrochloric acid to give the tetracarboxylic acid 4a and the mixed acid **4b**. At last, in order to generate the structure **4c,** carboxylic functions were converted into phosphonic functions using H₃PO₃/PCl₃ according to the method of Krüger and Bauer (Krüger and Bauer, 1972). The other strategy described in scheme II required preparation of protected bis-carboxymethylated amino ethyl bromide. In view to convenience of deprotecting ethyl esters by acid-catalysed hydrolysis, *N,N*-*bis*(ethylacetate)-2-bromoethyl-amine was prepared according to the Williams and Rapoport's procedure (Williams and Rapoport, 1994) with minor modifications. N-alkylation of **2d** with the branching group in a mixed solvent system (CH₃CN/EtOH) at 70°C gave **3d** in 60% yield. Acid-catalysed hydrolysis of the ester functions as well as nitriles is the more convenient method (Ornstein et al. , 1989), of hexacarboxylic acid **4d** preparation. 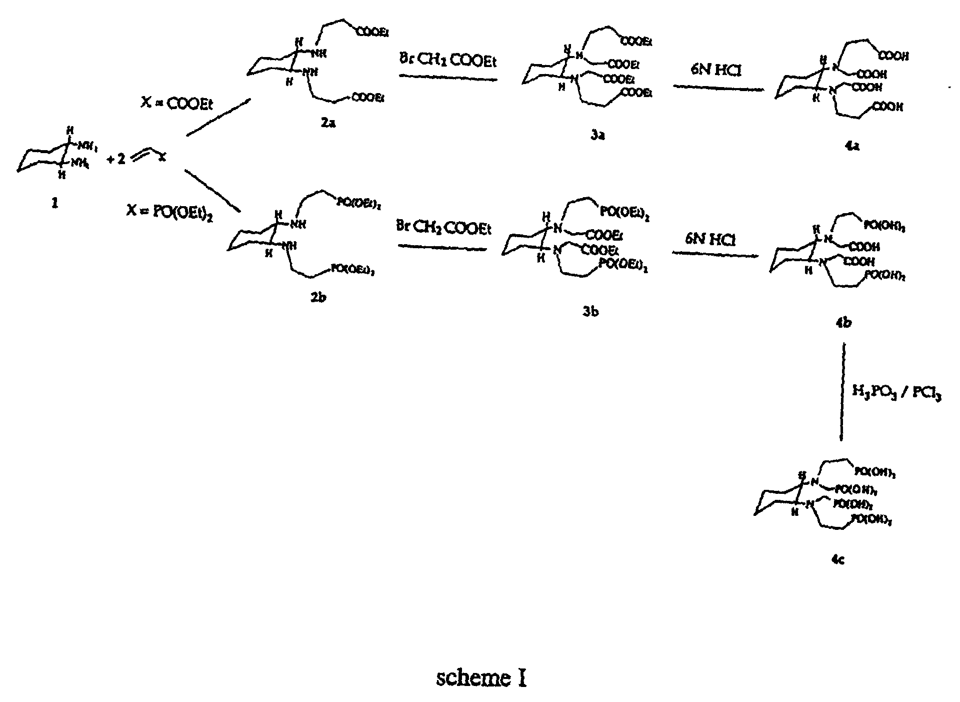 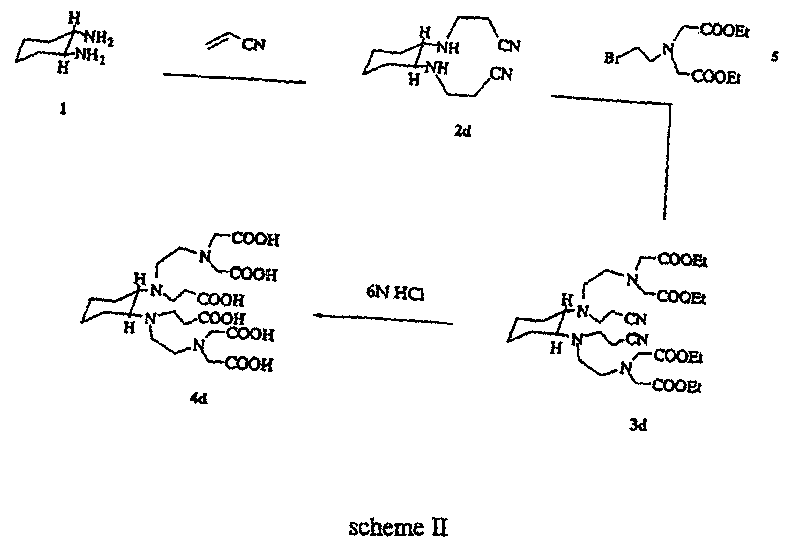
- In the case of certain diamines as *trans*-1,2-diaminocyclohexane **1,** the second route, as shown in schemes III, IV allowed the aminophosphonomethylation of amines protected by a methylene bridge between the two nitrogen atoms of **1**. This protecting group will subsequently provide for a different functionalization on the amine. The reaction of Kabachnick-field described and detailed by Baily and Burgada (Baily and Burgada, 1995), gave compound **6** which was prepared from paraformaldehyde and diethylphosphite in THF. **7** was obtained by removing the protecting group in acidic conditions.

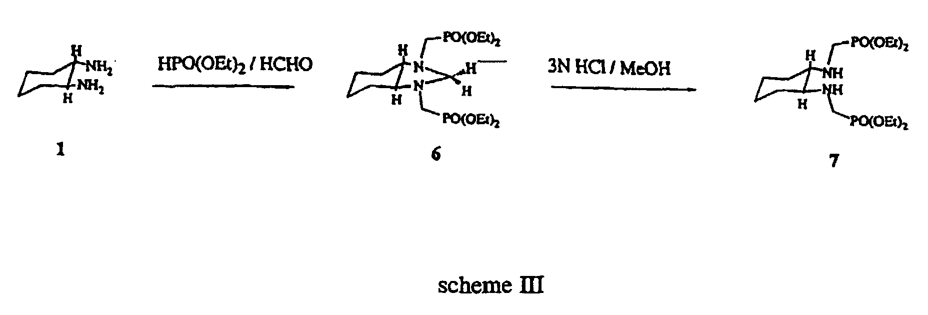

The monoalkylation or the dialkylation (see scheme IV), depending on the stoechiometry of the reaction gave respectively compounds **8** and **12.** The mixed acid **13** was obtained after hydrolysis of **12** in 6M hydrochloric acid and the hexaphosphonic acid **14** was prepared according to the method of Bauer and Kruger as described above. The synthesis of chelating agents **10** and **11** required an additional step which was the alkylation of **8** by ethyl bromoacetate to give the mixed ester **9.** Acid-catalysed hydrolysis gave **10** and **11** after reaction of conversion described all above.

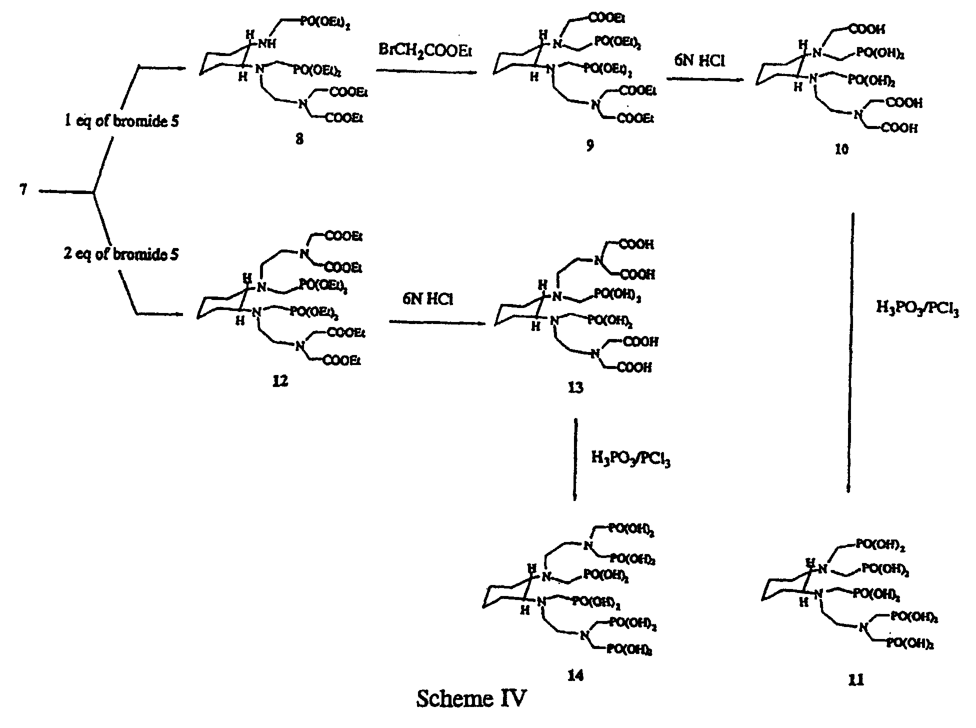

In conclusion, different non-functionalized ligands bearing aminophosphonate or aminocarboxylate chelate groups and mixed chelate groups were prepared and tested for their complexation properties with ¹⁵³Sm. The synthetic method described above was applied to the previously synthesized intermediate, the (1*R**, 2*R**, 4*S**)-4-acetamido-1,2-diaminocyclohexane, resulting in the synthesis of several polyaminocarboxylic acids, polyaminophosphonic acids and mixed semi-rigid functionalized ligands (BCA).

The different access routes to non-functionalized compounds described here were used without modifying the synthesis in order to obtain their functionalized homologues ready to be used in a coupling reaction as described in scheme V. We observed the influence of aminocarboxylic acid and aminophosphonic acid functions on the stability of the resulting complexes.

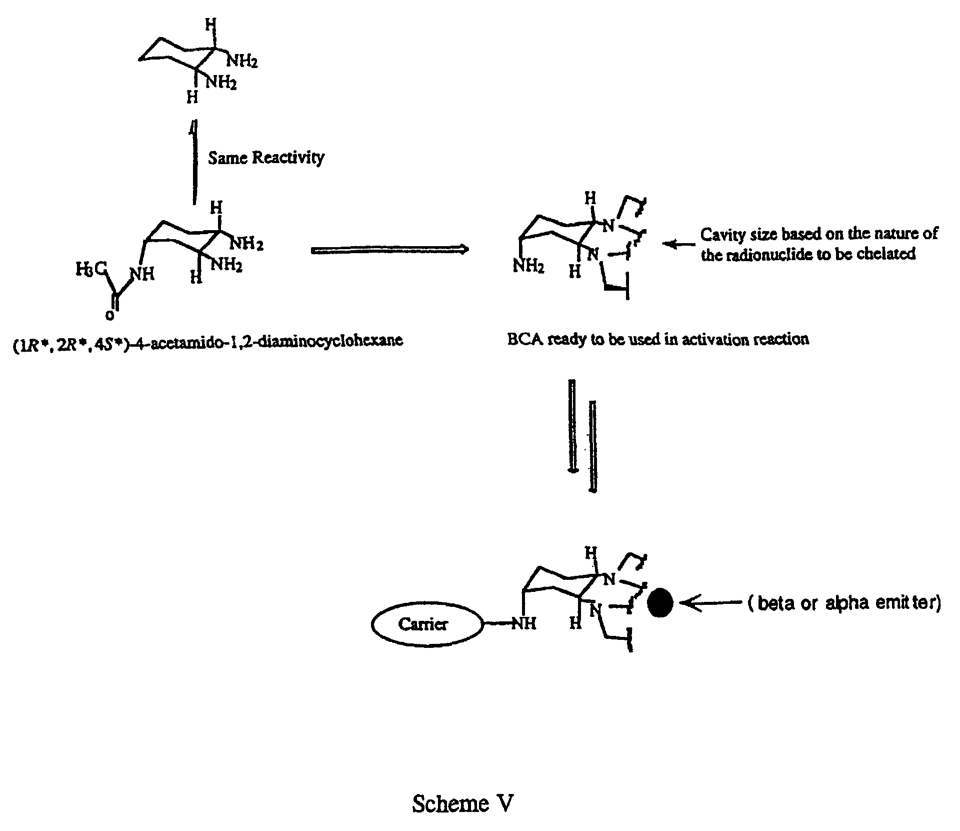

### Experimental

### General Procedures

All experiments were performed under nitrogen. Solvents were distilled prior to reactions. The primary chemicals used were commercial products (Sigma-Aldrich Company). Product purity and reaction progress were monitored on thin-layer chromatography (TLC) plates (60 F₂₅₄, Merck), and liquid chromatography was carried out on a silica gel column (Merck 60,70-230 mesh). TLC revelation was performed under UV light (254 nm) or by iodine.

### Nuclear Magnetic Resonance (MNR)

¹H and ¹³C NMR spectra were recorded on a Bruker AC 250 spectrometer (250 Mhz). Chemical shifts are reported in ppm to phosphoric acid as reference (85 % H₃PO₄ in heavy water), positive values being downfield.

Chemical shifts (d) are reported in ppm. Coupling constant J is reported in Hertz (Hz).

### Mass Spectrometry (MS)

MS spectra were recorded on a Mat Finnigan LCQ Ion Trap mass apparatus using the electrospray method in negative or positive mode.

### Starting Material

The bisphosphonate **6** was prepared in our laboratory according the synthesis procedure of Baily and Burgada with minor modifications.

### Synthesis and Specstroscopic data

### N,N'-[(2-ethoxycarbonyl)eth-1-yl]-trans-cyclohexane-1,2-diamine 2a:

To freshly distilled *trans*-1,2-diaminocyclohexane 1 (1 ml, 8.33 mmol) in 50 ml of ethanol was added vinyl propionate (1.50 ml, 13.7 mmol) in one portion. After stirring 20h at room temperature, the reaction mixture was concentrated by rotary evaporation to yield a pale yellow oil (2.6 g, 8.32 mmol, 100%) witch was used directly in the next step. ¹H NMR (CDCl₃): d 1.22 (t, 12H), 1.67 (m, 2H), 1.82 (m, 2H), 2.06 (m, 2H+2H), 2.43 (t, 4H), 2.67 (dt, 2H), 2.98 (dt, 2H), 4.10 (q, 4H). ¹³C NMR (CDCl₃): d 14.17, 24.31, 31.46, 35.34, 42.19, 60.23, 61.29, 172.69 , (M+H+): 315

***N,N'*-[(2-diethylphosphono)eth-1-yl]-*trans*-cyclohexane-1,2-diamine 2b:** To freshly distilled *trans*-1,2-diaminocyclohexane 1 (1 ml, 8.33 mmol) in 50 ml of ethanol was added diethyl vinyl phosphonate (2.80 ml, 18.21 mmol). The reaction mixture was allowed to stir at reflux during 15 hours. After removal the solvent under reduced pressure, the resulting oil was purified by column chromatography (silica gel, CH₂Cl₂-EtOH 1:1) to give 2.9 g of a limpid oil (6.65 mmol, 80%). ¹H NMR (CDCl₃): d 0.94 (m, 2H), 1.15 (m, 2H), 1.24 (t, 12H), 1.64 (m, 2H), 1.83-1.95 (m, 8H), 2.05 (m, 2H), 2.71 (dt, 2H), 2.97 (dt, 2H), 4.07 (dq, 8H). ¹³C NMR (CDCl₃): d 16.37, 16.46, 25.42 (J_{C-P} : 149 Hz), 28.20, 31.41, 40.47, 40.51, 61.29, 61.40, 61.50. (M+H⁺): 443

***N,N'*-[(2-cyano)eth-1-yl]-*trans*-cyclohexane-1,2-diamine 2d:** To freshly distilled *trans*-1,2-diaminocyclohexane 1 (1 ml, 8.33 mmol) in 50 ml of ethanol was added acrylonitryle (1.20 ml, 18.32 mmol). After stirring 20h at room temperature, the reaction mixture was concentrated by rotary evaporation to yield an pale yellow oil witch was purified by recristallisation in diethylether to give 1.40 g of a white solid (6.35 mmol, 78%): mp : 65°C
¹H NMR (CDCl₃): d 1.02 (m, 2H), 1.22 (m, 2H), 1.70-1.79 (m, 2H + 2H), 2.02-2.17 (m, 2H+2H), 2.49 (t, 4H), 2.80 (dt, 2H), 3.02 (dt, 2H). 13C NMR (CDCl₃): d (M+H+): 221

***N,N'*-[(2-ethoxycarbonyl)eth-1-yl]-*N,N'*-(ethylacetate)-*trans*-cyclohexane-1,2-diamine 3a:** To a solution of 2a (1 g; 3,.18 mmol) in 50 ml of freshly distilled CH3CN under nitrogen were added Na₂CO₃ (0.50 g; 3.01 mmol) and KI ( g; mmol). After stirring for 1 hour at 60°C, BrCH₂COOEt (1.80 ml; 7.15 mmol) was added dropwise. The reaction mixture was kept at this temperature over a period of 24 hours prior to cooling to room temperature, filtration and concentration under reduced pressure. The residue was taken up in CHCl₃ (200 ml) and washed with water. The organic layer was dried (Na₂SO₄) and concentrated under reduced pressure to give a yellow-brown oil. The crude product was purified by column chromatography (silica gel, CH₂Cl₂-EtOH 95:5). The fractions containing pure product were collected and dried to give an limpid oil (0.8 g; 1.56 mmol; 49 %). ¹H NMR (CDCl₃) d 1.10 (m, 4H), 1.26 (t, 12H), 1.91 (m, 2H), 2.00 (m, 2H), 2.51 (dt+m, 4H+2H(CH cycle)), 2.95 (dt, 4H), 3.39 (d, 4H), 4.12 (m, 8H). (M+H+): 515

***N,N'*-(ethylacetate)-*N,N'*-[(2-diethylphosphono)eth-1-yl]-*trans*-cyclohexane-1,2-diamine 3b:** The tetraester has been prepared as described above for compound from 1 g of compound 2b, Na₂CO₃ (0.70g, 6.60 mmol), KI (0.40, 2.40 mmol) and ethylbromoacetate (1.80 ml; 7.15 mmol). Purification by chromatography (SiO₂ (CH₂Cl₂-MeOH 99 : 1) gave 0.58 g of a pale yellow oil (0.94 mmol; 41%).¹H NMR (CDCl₃): d 1.11 (m, 4H), 1.25 (t, 6H), 1.29 (t, 12H), 1.70 (m, 2H), 1.85-2.10 (m, 4H+2H), 2.90 (t, 4H), 3.38 (d, 4H), 4.09 (m, 12H). ¹³C NMR (CDCl₃) d 14.11, 16.31, 16.41, 25.63, 26.03 (J_{C-P} : 135), 27.98, 44.86, 52.43, 60.26, 61.31, 61.41, 63.08, 172.46. (M+H⁺): 615

**General procedure for preparation of corresponding acids: *trans*-cyclohexane-1,2-diamine-*N,N'*-acetic-*N,N'*-propionic acid 4a and *trans*-cyclohexane-1,2-diamine-*N,N'*-acetic-*N,N'*-ethylphosphonic acid 4b:**

Compound 3a or 3b (1 g) was dissolved in 6N aqueous hydrochloric acid (12ml) and heated to reflux overnight. The refrigerant was removed, and the reaction mixture was kept at 70°C to dryness. An additional aqueous hydrochloric acid 6N (12 ml) was then added, and the solution was heated to dryness. the residue was taken up in MeOH and evaporated under reduced pressure. This step repeated twice gave the corresponding acid as an off-white solid, which was dried under vacuum and kept under nitrogen.

**Compound 4a:** ¹H NMR (D₂O): d 1.25-1.40 (m, 4H), 1.60-2.15 (m, 4H), 2.28 (m, 2H), 2.75 (t, 2H), 2.96 (t, 2H), 3.22 (m, 2H), 3.50-3.90 (m, 4H), 4.15 (s, 1H), 4.28 (s, 1H) ¹³C NMR (CDCl₃): d 25.66, 26.22, 28.87, 31.20, 31.99, 47.25, 54.93, 66.92, 175.03, 176.46 (M-H⁺): 373
**Compound 4b:** ¹H NMR (D₂O): d 1.05-1.40 (m, 4H), 1.65-2.15 (m, 4H), 2.90-3.25 (m, 6H), 3.45-3.65(m, 2H), 3.70-3.95 (m, 2H). ¹³C NMR (CDCl₃): d 25.66, 26.22, 28.87, 31.20, 31.99, 47.25, 54.93, 66.33, 176.73 (M-H⁺): 445

***trans*-cyclohexane-1,2-diamine-*N,N'*-ethylphosphonic-*N,N'*-methylphosphonic acid 4c:** A mixture of compound 4c (0.5 g; 1.12 mmol) and phosphorous acid (0.202 g; 2.46 mmol) in 10 ml of dry toluene was heated to 80°C and stirred for 30 min. PCl₃ ( 0.22 ml; 2.46 mmol) was then added dropwise, and the reaction mixture was kept at this temperature for 20 hours before being cooled to room temperature. The solvent was discarded and the residual product dissolved in a small volume of water. After filtration, the filtrate was evaporated to give a residue which was purified by precipitation in warm acetone and collected by filtration. The purification step was repeated twice to give 4c which was dried under vacuum and kept under nitrogen (0.460 g; 0.83 mmol; 74%). ¹H NMR (D₂O): d 1.15-1.65 (m, 4H), 1.75-2.10 (m, 2H), 2.15-2.40 (m, 6H), 3.00-3.60 (m, 10H). ¹³C NMR (CDCl₃): d (M-H+): 517.

***N,N-Bis*(ethylacetate)-2-bromoethyl-amine 5:** Bromide 5 was synthesised in our laboratory according the synthesis procedure of Williams and Rapoport with minor modifications concerning the bis N-alkylated ethanolamine synthesis. To a 4°C solution of ethanolamine (6ml; 0.1 mol) in 100 ml of dried acetonitrile was added dropwise ethylbromoacetate (7.4 ml; 66 mmol) over a period of 20 min during which time a large quantity of precipitate formed. The mixture was allowed to stir for 2 hours at this temperature. The white solid was removed by filtration and washed with a small quantity of acetonitrile. The filtrate was concentrated under reduced pressure. The resulting liquid was taken up in CHCl₃ (100mL) and washed with water. The organic layer was dried (Na₂SO₄) and concentrated under reduced pressure to give a liquid which was used directly in the next step (5.9 g; 25.32 mmol; 77%). The dialkylated ethanolamine and Ph3P (7.72 g; 27.9 mmol) were dissolved in CH₂Cl₂ (100mL). The mixture was cooled in an ice bath and vigorously stirred while NBS (4.96 g; 27.9 mmol) was added in small portions. After the solution was stirred at 0°C for two hours, evaporation of the solvent gave a semisolid which was tritured with ether and the resulting solid was separated by filtration, the filtrate was evaporated to give an oil which was purified by column chromatography (silica gel, CH₃Cl). (6.14 g; 20.7 mmol; 62% overall) ¹H NMR (CDCl₃): d 1.26 (m, 6H), 3.15 (t, 2H, J = 7.75 Hz), 3.44 (t, 2H, J = 7.75 Hz), 3.59 (s, 4H), 4.15 (q, 4H). ¹³C NMR (CDCl₃): d (M+H+): 297

***N,N'*-[(2-cyano)eth-1-yl]-N,N'-[*N",N"*-*bis*-(ethylacetate-2-aminoethyl)]-*trans*-cyclohexane-1,2-diamine 3d:** To a solution of compound 2d (1 g; 4.54 mmol) and bromide 5 (3 g; 10.13 mmol) in a mixed solvent system (CH₃CN-EtOH, 1:1) was added Na₂CO₃ (1.4 g; 13.20 mmol) and KI (0.75 g; 4.54 mmol). After stirring for 2 days at 70°C, the reaction mixture was filtered and concentrated under reduced pressure. The residue was taken up in CHCl₃ (200 ml) and washed with water. The organic layer was dried (Na₂SO₄) and concentrated under reduced pressure to give a yellow-brown oil. The crude product was purified by column chromatography (silica gel, CH₂Cl₂-BtOH 98:2). The fractions containing pure product were collected and dried to give an limpid oil (1.09 g; 1.68 mmol; 37%).¹H NMR (CDCl₃): d 1.13 (m, 4H), 1.27 (t, 12H), 1.73 (m, 2H), 1.86 (m, 2H), 2.30-2.85 (m, 4H+2H), 2.95 (m, 2H), 3.55 (s, 8H), 4.17 (m, 12H). ¹³C NMR (CDCl₃) d 14.25, 18.63, 25.79, 27.24, 47.35, 48.98, 53.78, 55.43, 60.54, 62.64, 119.49, 171.11. (M+H⁺): 652

***trans*-cyclohexane-1,2-diamine-*N,N'*-propionic-*N,N'*-[*N",N"*-*bis*-(2-aminoethyl)]-tetra-acetic acid 4d:** this hexaacid has been prepared as described above for compounds 4a & 4b from 1 g of the ester 3d and two volumes of 20 ml HCl (6N). ¹H NMR (D₂O): 1.20-2.00 (m, 10 H), 2.30 (m, 2H), 2.40-3.00 (m, 4H), 3.10-3.95 (m, 14 H), 4.20. (m, 4H) d ¹³C NMR (D₂O) : d 26.89, 30.49, 40.99, 52.00, 55.18, 55.47, 58.97, 165.50, 170.38. (M-H⁺): 547

***N,N'*-(diethylphosphono-methyl)-*trans*-cyclohexane-1,2-diamine 7:** was synthesised in our laboratory according the synthesis procedure of Baily and Burgada with minor modifications. Freshly distilled *trans*-1,2-diaminocyclohexane 1 (3.6 ml, 30 mmol) and diethyl phosphite (7.24 ml, 60 mmol) were dissolved in THF (40ml). The mixture was stirred at reflux and paraformaldehyde (2.8 g, 93 mmol) was added over a 30-min period and the reaction mixture was stirred at reflux for 4 hours. The solvent was evaporated to afford a residue which was taken up in CHCl₃. The organic layer was washed with brine (2*100 ml), dried and evaporated to leave a crude oil. A purification by column chromatography (silica gel, CH₂Cl₂-EtOH 96:4) gave 6 (9.2 g, 21.60 mmol, 72%). 6 was then dissolved in MeOH (40 ml) and 35% HCl (15 ml) was added. The mixture was stirred at 50°C overnight. MeOH was removed. the aqueous layer was adjusted to 50 ml with H₂O and then neutralized by HNaCO₃. bisphosphonate 7 was extracted by CHCl₃. Organic layers were collected, dried and evaporated to give 5.6 g of bisphosphonate 7 (13.52 mmol, 62%, 45% overall). ¹H NMR (CDCl₃): 1.10 (m, 2 H), 1.27 (t, 12H), 1.48 (m, 2H), 1.76 (m, 2 H), 2.13 (m, 2H), 2.98 (m, 2H), 3.10 (t, 2H), 3.35 (t, 2H), 4.12 (m, 8H). ¹³C NMR (CDCl₃): d: 16.36, 16.39, 16.45, 16.38, 24,01, 28.23, 39.67 (JP-C = 156 Hz), 60.74, 60.89, 63.16, 63.23, 63.26, 63.33. (M+H+): 415

***N,N'*-(diethylphosphono-methyl)-*N*-[*N",N"-bis*-(ethylacetate-2-aminoethyl)]-*trans*-cyclohexane-1,2-diamine 8:** The mixed ester has been prepared in a mixed solvent system (CH₃CN-H₂O, 1:1) as described above for compound 3d from bisphosphonate 6 (1 g, 2.41 mmol), Na₂HPO₄ (0.5 g; 3.50 mmol) and 1 equivalent of bromide 5 (0.71 g; 2.41 mmol). After stirring for 2 days at 70°C, the reaction mixture was filtered and concentrated under reduced pressure. The residue was taken up in CHCl₃ (200 ml) and washed with water. The organic layer was dried (Na₂SO₄) and concentrated under reduced pressure to give a yellow-brown oil witch was used directly in the next step. ¹H NMR: 1.00-1.20 (m, 4 H), 1.25 (t, 6H), 1.31 (t,12H), 1.72 (m, 2H), 2.04 (m, 2H), 2.23 (m, 1 H), 2.70-2.95 (m, 6H), 3.12 (m, 2H), 3.65 (s, 4H), 4.15 (m, 18H).

***N,N'*-(diethylphosphono-methyl)-*N'*-(ethylacetate)-*N*-[*N",N"-bis*-(ethylacetate-2-aminoethyl)]-*trans*-cyclohexane-1,2-diamine 9:** The tetraester is prepared as described above for compounds 3a & 3b from compound 8, Na₂CO₃, KI and ethylbromoacetate (1 equivalent). Purification by chromatography (SiO₂) gave a oil.

***N,N'*-(diethylphosphono-methyl)-*N,N'*-[*N",N"-bis*-(ethylacetate-2-aminoethyl)]-*trans*-cyclohexane-1,2-diamine 12:** The mixed ester is prepared as described above for compound 3d from bisphosphonate 7 (1g, 2.41 mmol), Na₂HPO₄ (1 g, 7.04 mmol) and 2 equivalents of bromide 5 1.80 g, 6.08 mmol). Purification by chromatography (SiO₂ (CH₂Cl₂-MeOH 95 : 5) gave a pale yellow oil (0.59 g, 0.70 mmol, 29%). ¹H NMR: 1.16 (m, 4H), 1.25 (t, 12H), 1.33 (t, 12H), 1.69 (m, 2H), 1.86 (m, 2H + 2H), 2.70-3.50 (m, 8H + 2H + 2H), 3.57 (s, 8H), 4.12 (m, 16H). (M+H+): 847

***trans*-cyclohexane-1,2-diamine-*N,N'*-methylphosphonic-*N*-*acetic*-,*N'*-[*N",N"*-*bis*-(2-aminoethyl)]-tetra-acetic acid 10 & *trans*-cyclohexane-1,2-diamine-*N,N'*-methylphosphonic-*N,N'-*[*N",N"*-*bis*-(2-aminoethyl)]-tetra-acetic acid 13 :** Those compounds are prepared as described above for compound 4a, 4b and 4d.

**Compound 13:** ¹H NMR (D₂O): 1.26 (m, 2 H), 1.45 (m, 2H), 1.75-2.00 (m, 4H), 2.80 (m, 2H), 3.00-3.70 (m, 12H), 4.10 (br s, 8H). (M-H+): 619

***trans*-cyclohexane-1,2-diamine-*N,N,N'*-methylphosphonic-*N'*-[*N",N"-bis*-(2-aminoethyl)]-di-methylphosphonic acid 11 & *trans*-cyclohexane-1,2-diamine-*N,N'*-methylphosphonic-*N,N'*-[*N",N"*-*bis*-(2-aminoethyl)]-tetra-methylphosphonic acid 14 :** Those compounds are prepared as described above for compound 4c.

### 153Sm complexation studies

Complexation studies with Samarium 153 were performed on the two following chelating agents 10 (AL 247) and 13 (AL 245)

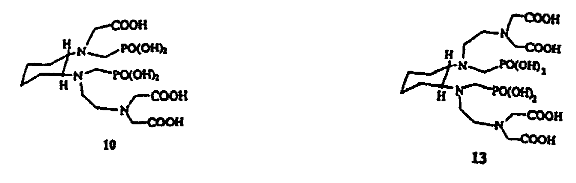

Radiochemistry purity was measured on ITLC-SG chromatographic profiles. Radioactivity was quantified using a Phosphorimager 445SI apparatus.

Samarium 153 was furnished under 153SmCl₃ form in HCl 0,04N with a 5,2 GBq/ml volumic activity and a 40 GBq/mg specific activity.

They were tested for their complexation properties by using an excess of 10 to 50 equivalents of chelating agent.

Competition studies were performed against EDTMP according to the following method :
- first step : 50 equivalents of one of the chelating agents and a fixed amount of 153Sm were incubated at 37°C during 3 hours in order to form the 153Sm-CA complex (CA = Chelating Agent),
- second step : 50 equivalents of EDTMP were added to the previous solution and kept 3h at 37°C in order to measure the decomplexation. Another measure was performed 72h after to ensure a complete decomplexation possibility.

| Results : | | |
|---|---|---|
| | % of non decomplexed 153Sm-CA | |
| chelating agent | after 3h | after 72h |
| 10 (AL 247) | 100 | 67 |
| 13 (AL 245) | 100 | 100 |

AL 247 and AL 245 present very good complexation properties for 153Sm and in any cases better than EDTMP

Furthermore, stability was performed on AL 245 in human serum media at 37°C at different time and showed no loss of 153Sm from AL 245 at either 24, 48, 72 and 96h.

### References

- T. Baily and R. Burgada, *Phosphorus, Sulfur and Silicon*., 1995, **101,** 131.
- M.Bardies, P. Thedrez, J.F. Gestin, B.M. Marcille, D. Guerreau, A. Faivre-Chauvet, M. Mahé, C. Sai-Maurel and J.F. Chatal, *Int. J. Cancer*, 1992, **50,** 984.
- R. J. Bergeron, P.S. Burton, K.A. McGovern aaaand S.J. Kline, *Synthesis.* 1981, 732.
- M. W. Brechbiel, O. A. Gansow, C. G. Pippin, R. D. Rogers, and R. P. Planalp, Inorg. Chem. 1996, 35, 6343-6348.
- J-F. Gestin, E. Benoist, A. Loussouarn, A.K. Mishra, A. Faivre-Chauvet and J-F. Chatal, *New J. of Chem*., 1997, **21,** 1021.
- W. F. Goeckeler, B. Edwards,W.A. Volkert, R.A. Holmes, J. Simon and D. Wilson, *J. Nucl. Med*., 1987, **28**, 495.
- F. Krüger and L. Bauer, *Chem.Ztg*., 1972, **36,** 691.
- A. Loussouarn, M. Duflos, E. Benoist, J-F. Chatal, G. Le Baut and J-F. Gestin, *J. Chem. Soc. Perkin Trans*., *1998*, **1,** 237.
- C.F. Meares, M.J. Mc Call, D.T. Reardan, D.A. Goodwin, C.I. Diamanti and M. McTigue, *Anal. Chem*., 1984, **142,** 68.
- R.C. Mease, S.C. Srivastava, G.E. Meinken, J-F. Gestin and Z. Steplewski, *J. Nucl. Med*., 1990, **31,** 896.
- P. L. Ornstein, J. M. Schaus, J. W. Chambers, D. L. Huser, J. D. Leander, D. T. Wong, J. W. Paschal, N. D. Jones and, J. B. Deeter, *J. Med*. *Chem*. 1989, **32,** 827.
- D.Parker, *Chem. Soc. Review,* 1990, **19,** 271.
- P.A. Schubiger, R. Alberto and A. Smith, *Bioconjugate Chem*., 1996, **7,** 165.
- R. Stein, D. M. Goldenderg, S. R. Thorpe, A. Basu and M. J. Mattes, *Cancer Research*, 1995, **55,** 3132.
- M. Studer and C.F. Meares, *Bioconjugate Chem*., 1992, **3,** 420.
- M. A. Williams and H. Rapoport, *J. Org. Chem*., 1994, **59,** 3616.

## Claims

1. Compounds of the following formula (I) : 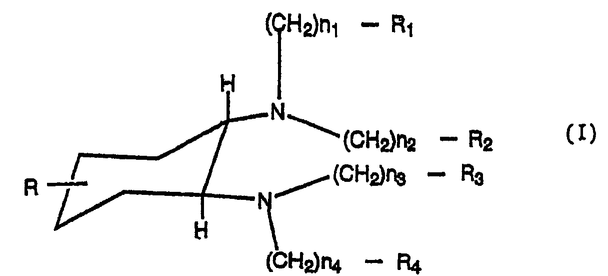 in which :
- n₁, n₂, n₃ and n₄, independently from each other, represent an integer from 1 to 5,
- R₁, R₂, R₃ and R₄, independently from each other, represent:
. -COOH,
. -PO(OH)₂, 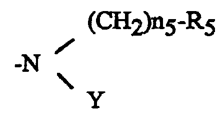
wherein n₅ represents an integer from 1 to 5, R₅ represents -COOH or -PO(OH)₂, and Y represents H or a group -(CH2)n₆-R₆ in which n₆ represents an integer from 1 to 5, and R₆ represents -COOH or -PO(OH)₂,
provided that at least one of R₁, R₂, R₃ or R₄ represents a group 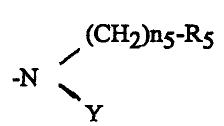 such as defined above,
- R represents :
. H, or -NHCOCH₃, or
. a group carrying a function liable to bind, if necessary via a binding site, to molecules which are able to bind specifically with epitopes located at the surface of the cells of the organism, or to chemical or biological compounds located at the surface of a solid carrier, or
. a group carrying a function linked, if necessary via a binding site, to molecules as defined above, the two following compounds, CDTPA and CTTHA, being excluded :

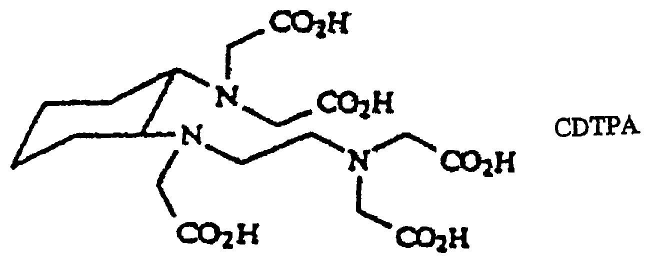

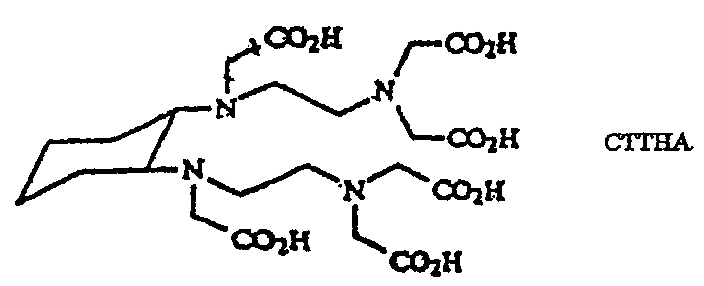

2. Compounds according to claim 1, **characterized in that** :
- n₁, n₂, n₃, n₄, n₅, and n₆, independently from each other, represent an integer from 1 to 3,
- molecules which are able to bind specifically with epitopes located at the surface of the cells of the organism, or to chemical or biological compounds located at the surface of a solid carrier, are antibodies, haptens or peptides.

3. Compounds according to claim 1 or 2, **characterized in that** :
- when R₁, R₂, R₃ or R₄ represents -COOH or -PO(OH)₂, then n₁, n₂, n₃ or n₄ represents 1 respectively,
- when R₁, R₂, R₃ or R₄ represents a group 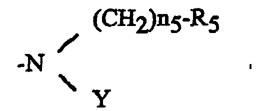 then n₁, n₂, n₃ or n₄ represents 2 or 3 respectively,
- n₅, and optionally n₆, represents 1.

4. Compounds according to anyone of claims 1 to 3, **characterized in that** at least one of R₁, R₂, R₃ and R₄, represent a group 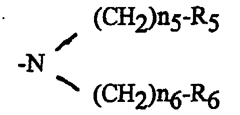 wherein n₅, n₆, R₅ and R₆ are defined in claims 1 to 3.

5. Compounds according to claim 4, **characterized in that** at least two of R₁, R₂, R₃ and R₄, represent a group 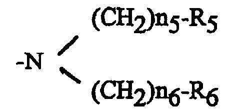 wherein n₅, n₆, R₅ and R₆ are defined in claims 1 to 3.

6. Compounds according to anyone of claims 1 to 5, **characterized in that** R represents a group carrying a function liable to bind, if necessary via a binding site, to molecules as defined in claims 1 or 2, R being a group chosen among all the coupling functions for vector or solid support binding.

7. Compounds according to anyone of claims 1 to 5, **characterized in that** R represents a group carrying a function linked, if necessary via a binding site, to molecules as defined in claims 1 or 2.

8. Compounds according to anyone of claims 1 to 7 of the following formula (III): 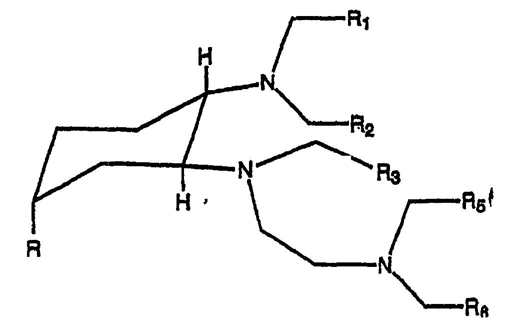 in which R₁, R₂, R₃, R₅ and R₆ independently from each other represent -COOH or -PO(OH)₂, and R is a group as defined in claims 1 to 7.

9. Compounds according to claim 8, of formula (III) wherein :
. R₁ = R₅ = R₆ = COOH and R₂, = R₃ = PO(HO)₂, or
. R₁ = R₂ = R₃ = R₅ = R₆ = COOH, or
. R₁ = R₂ = R₃ = R₅ = R₆ = PO(OH)₂.

10. Compounds according to anyone of claims 1 to 7, of the following formula (IV): 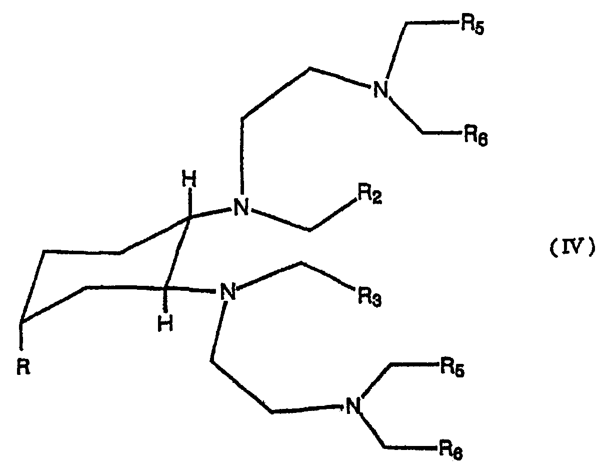 wherein R₂, R₅ and R₆, independently form each other, represent -COOH or -PO(OH)₂, and R is a group as defined in claims 1 to 7.

11. Compounds according to claim 10 of formula (IV) wherein :
. R₂ = R₃ = PO(OH)₂, and R₅ = R₆ = COOH, or
. R₂ = R₃ = R₅ = R₆ = COOH, or
. R₂ = R₃ = R₅ = R₆ = PO(OH)₂.

12. Complexes between a compound according to anyone of claims 1 to 11, and a radioactive element.

13. Complexes according to claim 12, **characterized in that** said radioelements are α or β emitter radiometals.

14. Complexes according to claim 13, **characterized in that** the compound is chosen among those defined in anyone of claims 7 to 11.

15. Complexes according to claim 13 or 14, wherein the group R comprises :
- an antibody (polyclonal or monoclonal) liable to recognize and to bind to specific epitopes on the surface of specific cells of the organism,
- or an hapten, i.e. a non-immunogenic molecule of low MW capable of inducing the production of antibodies against itself, said hapten being liable to recognize and to bind to one or several molecules already bound, in a first step of the treatment, to epitopes on the surface of specific cells in the organism,
- or a peptide resulting from the association of different amino acids and liable to recognize and to bind to specific epitopes on the surface of specific cells of the organism.

16. Pharmaceutical compositions **characterized in that** they comprise an effective amount of a compound according to anyone of claims 1 to 11, or a complex according to anyone of claims 13 to 15, in association with a suitable pharmaceutical carrier.

17. Use of a complex according to anyone of claims 13 to 15, for the manufacture of a medicament for radioimmunotherapy, such as for the treatment of cancers, and more particularly for the treatment against metastas proliferation.

18. Complexes according to claim 12, **characterized in that** the radioelements are γ emitter radiometals.

19. Complexes according to claim 18, **characterized in that** the compound is chosen among those defined in anyone of claims 7 to 11, and wherein the group R comprises :
- an polyclonal or monoclonal antibody liable to recognize and to bind to specific epitopes on the surface of specific cells of the organism,
- or an hapten, i.e. a non-immunogenic molecule of low MW capable of inducing the production of antibodies against itself, said hapten being liable to recognize and to bind to one or several molecules already bound, in a first step of the method of diagnosis, to epitopes on the surface of specific cells in the organism,
- or a peptide resulting from the association of different amino acids and liable to recognize and to bind to specific epitopes on the surface of specific cells of the organism.

20. Complexes according to claim 17 or 18, for carrying out diagnosis methods such as radioimmunoscintigraphy.

21. Use of a compound of formula (I) as defined in claims 1 to 11, including CDTPA and CTTHA, for the manufacture of a medicament useful as antalgic, or for the treatment of pathologies where ionic imbalances occur, or against the formation of stones in the organism.

22. Use of a compound of formula (I) as defined in claims 1 to 11, including CDTPA and CTTHA, for carrying out a process for the detoxication of polluted medium, such as liquid phases polluted by bivalent or trivalent metals radioactives or not.

23. Use of a compound of formula (I) as defined in claims 1 to 11, including CDTPA and CTTHA, for carrying out a process for the radionuclides purification, said compound being bound to a solid phase.

24. Use of a compound of formula (I) as defined in claims 1 to 11, including CDTPA and CTTHA, for carrying out a bone scintigraphy, in particular in the frame of the diagnosis of osteoarticular pathology, particularly in bone cancer extension balance.

## Patentansprüche

1. Verbindungen der folgenden Formel (I): 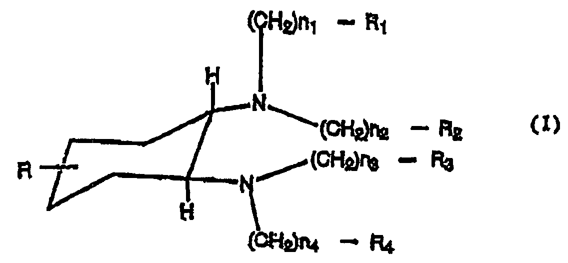 in welchen:
- n₁, n₂, n₃, und n₄ unabhängig voneinander eine ganze Zahl von 1 bis 5 darstellen,
- R₁, R₂, R₃ und R₄ unabhängig voneinander darstellen:
. -COOH,
. -PO(OH)₂, 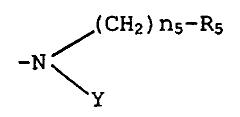
worin n₅ eine ganze Zahl von 1 bis 5 darstellt, R₅ -COOH oder -PO(OH)₂ darstellt, und Y H oder eine Gruppe -(CH₂)n₆-R₆ darstellt, in welcher n₆ eine ganze Zahl von 1 bis 5 und R₆ -COOH oder -PO(OH)₂ darstellt,
vorausgesetzt, dass wenigstens eines von R₁, R₂, R₃ oder R₄ eine Gruppe darstellt 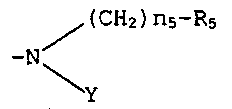 wie vorher definiert,
- R stellt dar:
. H, oder -NHCOCH₃, oder
. eine Gruppe, die eine Funktion trägt geeignet dafür, wenn notwendig über eine Bindungsstelle, an Moleküle zu binden, die spezifisch an Epitope, lokalisiert auf der Oberfläche von Zellen eines Organismus, oder an chemische oder biologische Verbindungen, lokalisiert auf der Oberfläche eines festen Trägers, binden können, oder
. eine Gruppe, welche eine Funktion trägt ,die mit wie vorher definierten Moleküle verbunden ist, wenn notwendig über eine Bindungsstelle,
wobei die folgenden Verbindungen, CDTPA und CTTHA, ausgeschlossen sind

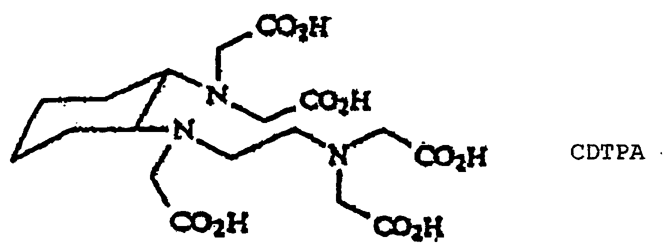

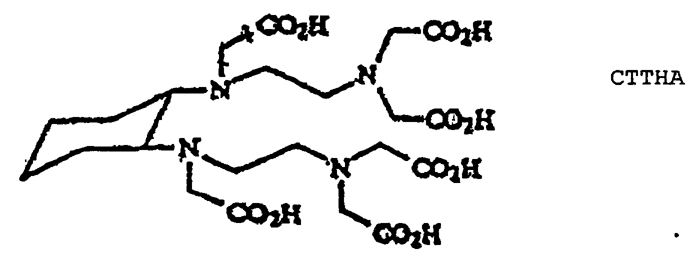

2. Verbindungen nach Anspruch 1, **dadurch gekennzeichnet, dass**:
- n₁, n₂, n₃, n₄, n₅ und n₆ unabhängig voneinander eine ganze Zahl von 1 bis 3 darstellen,
- die Moleküle, welche spezifisch an Epitope, lokalisiert auf der Oberfläche von Zellen eines Organismus, oder an chemische oder biologische Verbindungen, lokalisiert auf der Oberfläche eines festen Trägers, binden können, Antikörper, Haptene oder Peptide sind.

3. Verbindungen nach Anspruch 1 oder 2, **dadurch gekennzeichnet, dass**:
- falls R₁, R₂, R₃ oder R₄ -COOH oder -PO(OH)₂ darstellen, dann stellen n₁, n₂, n₃ bzw. n₄ 1 dar,
- falls R₁, R₂, R₃ oder R₄ eine Gruppe darstellen 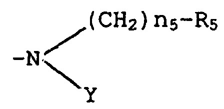 dann stellen n₁, n₂, n₃ bzw. n₄ 2 oder 3 dar,
- n₅, und wahlweise n₆, 1 darstellt.

4. Verbindungen nach einem der Ansprüche 1 bis 3, **dadurch gekennzeichnet, dass** wenigstens eines von R₁, R₂, R₃ und R₄ eine Gruppe darstellt 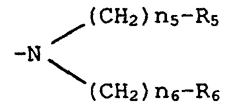 worin n₅, n₆, R₅ und R₆ wie in den Ansprüchen 1 bis 3 definiert sind.

5. Verbindungen nach Anspruch 4, **dadurch gekennzeichnet, dass** wenigstens zwei von R₁, R₂, R₃ und R₄ eine Gruppe darstellen 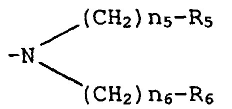 worin, n₅, n₆, R₅ und R₆ wie in den Ansprüchen 1 bis 3 definiert sind.

6. Verbindungen nach einem der Ansprüche 1 bis 5,
**dadurch gekennzeichnet, dass** R eine Gruppe darstellt, die eine Funktion trägt, die dafür geeignet ist, an wie in den Ansprüchen 1 oder 2 definierte Moleküle zu binden, wenn notwendig über eine Bindungsstelle, wobei R eine Gruppe ausgewählt aus allen Kopplungsfunktionen für Vektor- oder Festträgerbindungen ist.

7. Verbindungen nach einem der Ansprüche 1 bis 5, **dadurch gekennzeichnet, dass** R eine Gruppe darstellt, die eine Funktion trägt, die mit wie in den Ansprüchen 1 oder 2 definierten Molekülen verbunden ist, wenn notwendig über eine Bindungsstelle.

8. Verbindungen nach einem der Ansprüche 1 bis 7, der folgenden Formel (III) : 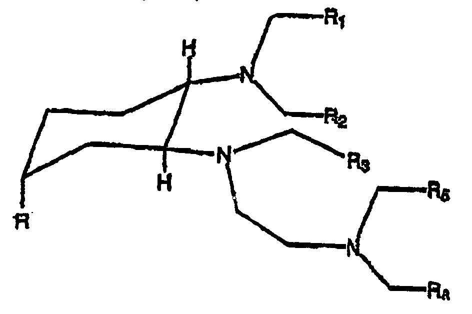 in welchen R₁, R₂, R₃, R₅ und R₆ unabhängig voneinander -COOH oder -PO(OH)₂ darstellen, und R eine Gruppe wie in den Ansprüchen 1 bis 7 definiert ist.

9. Verbindungen nach Anspruch 8 der Formel (III) worin:
. R₁ = R₅ = R₆ = COOH, und R₂ = R₃ = PO(HO)₂, oder
. R₁ = R₂ = R₃ = R₅ = R₆ = COOH, oder
. R₁ = R₂ = R₃ = R₅ = R₆ = PO(HO)₂.

10. Verbindungen nach einem der Ansprüche 1 bis 7, der folgenden Formel (IV) : 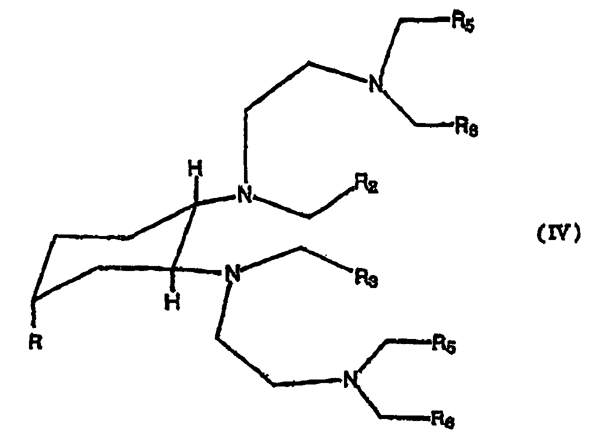 worin R₂, R₅ und R₆ unabhängig voneinander -COOH oder -PO(OH)₂ darstellen, und R eine Gruppe wie in den Ansprüchen 1 bis 7 definiert ist.

11. Verbindungen nach Anspruch 10 der Formel (IV) worin:
. R₂ = R₃ = PO(HO)₂, und R₅ = R₆ = COOH, oder
. R₂ = R₃ = R₅ = R₆ = COOH, oder
. R₂ = R₃ = R₅ = R₆ = PO(HO)₂.

12. Komplexe zwischen einer Verbindung nach einem der Ansprüche 1 bis 11 und einem radioaktiven Element.

13. Komplexe nach Anspruch 12, **dadurch gekennzeichnet, dass** die radioaktiven Elemente α- oder β-Strahlung emittierende, radioaktive Metalle sind.

14. Komplexe nach Anspruch 13, **dadurch gekennzeichnet, dass** die Verbindung aus denen in einem der Ansprüche 7 bis 11 definierten ausgewählt ist.

15. Komplexe nach Anspruch 13 oder 14, wobei die Gruppe R umfasst:
- einen Antikörper (polyklonal oder monoklonal), geeignet dafür spezifische Epitope auf der Oberfläche der spezifischen Zellen des Organismus zu erkennen und zu binden,
- oder ein Hapten, d.h. ein nicht-immunogenes Molekül mit niedrigem Mw, welches die Produktion von Antikörpern gegen sich selbst induzieren kann, wobei das Hapten dafür geeignet ist ein oder mehrere bereits in einem ersten Schritt der Behandlung an Epitope auf der Oberfläche spezifischer Zellen in dem Organismus gebundene Moleküle zu erkennen und daran zu binden,
- oder ein Peptid, resultierend aus der Verbindung verschiedener Aminosäuren, und welches dafür geeignet ist spezifische Epitope auf der Oberfläche spezifischer Zellen des Organismus zu erkennen und daran zu binden.

16. Pharmazeutische Zusammensetzungen, **dadurch gekennzeichnet, dass** sie eine wirksame Menge einer Verbindung nach einem der Ansprüche 1 bis 11 oder einen Komplex nach einem der Ansprüche 13 bis 15 in Verbindung mit einem geeigneten pharmazeutischen Träger umfassen.

17. Verwendung eines Komplexes nach einem der Ansprüche 13 bis 15 für die Herstellung eines Medikaments für die Radioimmuntherapie, wie etwa für die Behandlung von Krebsarten und insbesondere für eine Behandlung gegen Metastasenproliferation.

18. Komplexe nach Anspruch 12, **dadurch gekennzeichnet, dass** die radioaktiven Elemente γ-Strahlung emittierende, radioaktive Metalle sind.

19. Komplexe nach Anspruch 18, **dadurch gekennzeichnet, dass** die Verbindung aus denen in einem der Ansprüche 7 bis 11 definierten ausgewählt ist, und worin die Gruppe R umfasst:
- einen polyklonalen oder monoklonalen Antikörper geeignet dafür spezifische Epitope auf der Oberfläche spezifischer Zellen des Organismus zu erkennen und daran zu binden,
- oder ein Hapten, d.h. ein nicht-immunogenes Molekül mit niedrigem Mw, welches die Produktion von Antikörpern gegen sich selbst induzieren kann, wobei das Hapten dafür geeignet ist ein oder mehrere bereits in einem ersten Diagnoseschritt an Epitope auf der Oberfläche spezifischer Zellen in dem Organismus gebundene Moleküle zu erkennen und daran zu binden,
- oder ein Peptid, resultierend aus der Verbindung verschiedener Aminosäuren, und welches geeignet dafür ist, spezifische Epitope auf der Oberfläche spezifischer Zellen des Organismus zu erkennen und daran zu binden.

20. Komplexe nach Anspruch 17 oder 18, zur Durchführung von Diagnoseverfahren wie etwa der Radioimmunszintigraphie.

21. Verwendung einer Verbindung der Formel (I) wie in einem der Ansprüche 1 bis 11 definiert, einschließlich CDTPA und CTTHA, für die Herstellung eines Medikaments verwendbar als Analgetikum oder für die Behandlung von Krankheiten in denen Ionen-Ungleichgewichte auftreten oder gegen die Bildung von Steinen in dem Organismus.

22. Verwendung einer Verbindung der Formel (I) wie in einem der Ansprüche 1 bis 11 definiert, einschließlich CDTPA und CTTHA, für die Durchführung eines Verfahrens der Entgiftung eines verunreinigten Mediums, wie etwa flüssige Phasen, verunreinigt durch zweiwertige oder dreiwertige radioaktive oder nicht-radioaktive Metalle.

23. Verwendung einer Verbindung der Formel (I), wie in einem der Ansprüche 1 bis 11 definiert, einschließlich CDTPA und CTTHA, für die Durchführung eines Verfahrens der Radionuklidreinigung, wobei die Verbindung an die stationäre Phase gebunden ist.

24. Verwendung einer Verbindung der Formel (I) wie in einem der Ansprüche 1 bis 11 definiert, einschließlich CDTPA und CTTHA, für die Durchführung einer Knochenszintigraphie, besonders im Rahmen der Diagnose der osteoartikulären Pathologie, insbesondere des Gleichgewichts der Knochenkrebsausdehnung.

## Revendications

1. Composés de formule (I) suivante : 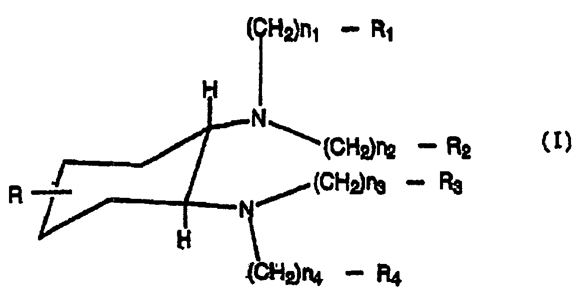 dans laquelle :
- n₁, n₂, n₃ et n₄, indépendamment les uns des autres, représentent un nombre entier de 1 à 5,
- R₁, R₂, R₃ et R₄, indépendamment les uns des autres, représentent :
. -COOH,
. -PO(OH)₂, 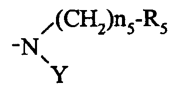
dans lequel n₅ représente un nombre entier de 1 à 5, R₅ représente -COOH ou - PO(OH)₂, et Y représente H ou un groupe -(CH₂)n₆-R₆ dans lequel n₆ représente un nombre entier de 1 à 5, et R₆ représente -COOH ou -PO(OH)₂,
sous réserve qu'au moins un de R₁, R₂, R₃ ou R₄ représente un groupe 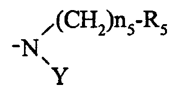 tel que défini ci-dessus,
- R représente :
. H, ou -NHCOCH₃, ou
. un groupe portant une fonction susceptible de se lier, si nécessaire via un site de liaison, à des molécules qui sont capable de se lier de manière spécifique à des épitopes situés à la surface des cellules de l'organisme, ou à des composés chimiques ou biologiques situés à la surface d'un support solide, ou
. un groupe portant une fonction liée, si nécessaire via un site de liaison, à des molécules telles que définies ci-dessus,
les deux composés suivants, CDTPA et CTTHA, étant exclus :

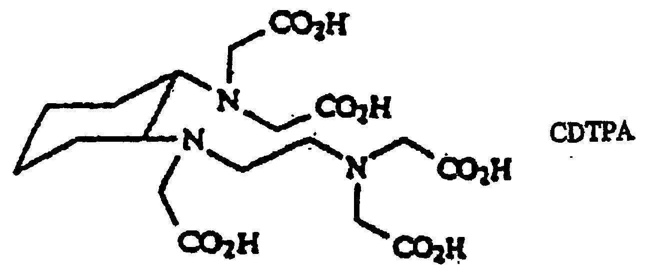

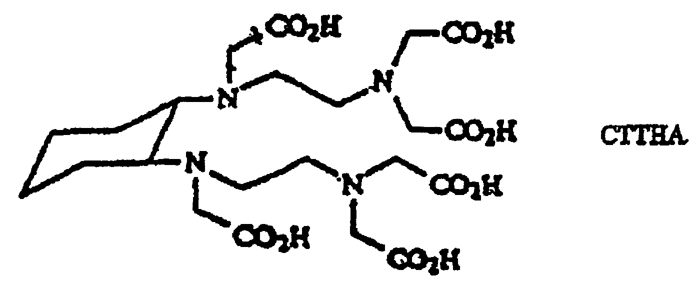

2. Composés selon la revendication 1, **caractérisés en ce que** :
- n₁, n₂, n₃, n₄, n₅, et n₆, indépendamment les uns des autres, représentent un nombre entier de 1 à 3,
- les molécules qui sont capables de se lier de manière spécifique à des épitopes situés à la surface des cellules de l'organisme, ou à des composés chimiques ou biologiques situés à la surface d'un support solide, sont des anticorps, des haptènes ou des peptides.

3. Composés selon la revendication 1 ou 2, **caractérisés en ce que** :
- lorsque R₁, R₂, R₃ ou R₄ représente -COOH ou -PO(OH)₂, alors n₁, n₂, n₃ ou n₄ représente 1 respectivement,
- lorsque R₁, R₂, R₃ ou R₄ représente un groupe 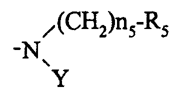 alors n₁, n₂, n₃ ou n₄ représente 2 ou 3 respectivement,
- n₅, et éventuellement n₆, représente 1.

4. Composés selon l'une des revendications 1 à 3, **caractérisés en ce qu'**au moins un de R₁, R₂, R₃ et R₄, représente un groupe 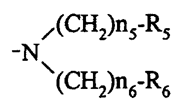 dans lequel n₅, n₆, R₅ et R₆ sont définis dans les revendications 1 à 3.

5. Composés selon la revendication 4, **caractérisés en ce qu'**au moins deux de R₁, R₂, R₃ et R₄, représentent un groupe 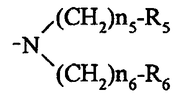 dans lequel n₅, n₆, R₅ et R₆ sont définis dans les revendications 1 à 3.

6. Composés selon l'une des revendications 1 à 5, **caractérisés en ce que** R représente un groupe portant une fonction susceptible de se lier, si nécessaire via un site de liaison, à des molécules telles que définies dans la revendication 1 ou 2, R étant un groupe choisi parmi l'ensemble des fonctions de couplage pour la liaison à un vecteur ou à un support solide.

7. Composés selon l'une des revendications 1 à 5, **caractérisés en ce que** R représente un groupe portant une fonction liée, si nécessaire via un site de liaison, à des molécules telles que définies dans les revendications 1 ou 2.

8. Composés selon l'une des revendications 1 à 7 de formule (III) suivante : 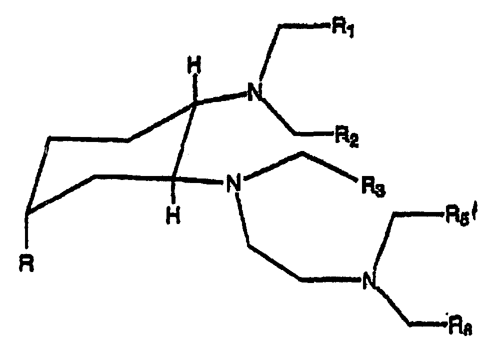 dans laquelle R₁, R₂, R₃, R₅ et R₆ indépendamment les uns des autres représentent -COOH ou -PO(OH)₂, et R est un groupe tel que défini dans les revendications 1 à 7.

9. Composés selon la revendication 8, de formule (III) dans laquelle :
. R₁ = R₅ = R₆ = COOH, et R₂ = R₃ = PO(OH)₂, ou
. R₁ = R₂ = R₃ = R₅ = R₆ = COOH, ou
. R₁ = R₂ = R₃ = R₅ = R₆ = PO(OH)₂.

10. Composés selon l'une des revendications 1 à 7, de formule (IV) suivante : 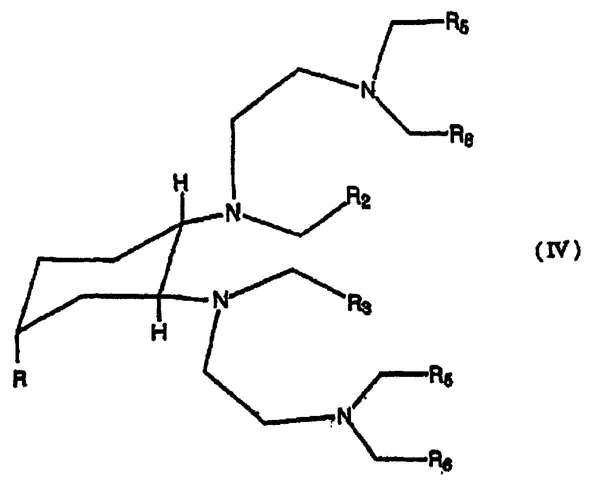 dans laquelle R₂, R₅ et R₆, indépendamment les uns des autres, représentent -COOH ou -PO(OH)₂, et R est un groupe tel que défini dans les revendications 1 à 7.

11. Composés selon la revendication 10 de formule (IV) dans laquelle :
. R₂ = R₃ = PO(OH)₂, et R₅ = R₆ = COOH, ou
. R₂ = R₃ = R₅ = R₆ = COOH, ou
. R₂ = R₃ = R₅ = R₆ = PO(OH)₂.

12. Complexes entre un composé selon l'une des revendications 1 à 11, et un élément radioactif.

13. Complexes selon la revendication 12, **caractérisés en ce que** lesdits radioéléments sont des radiométaux émetteurs α ou β.

14. Complexes selon la revendication 13, **caractérisés en ce que** le composé est choisi parmi ceux définis dans l'une des revendications 7 à 11.

15. Complexes selon la revendication 13 ou 14, dans lesquels le groupe R comprend :
- un anticorps (polyclonal ou monoclonal) susceptible de reconnaître et de se lier à des épitopes spécifiques à la surface de cellules spécifiques de l'organisme,
- ou un haptène, c'est-à-dire une molécule non immunogène de faible PM capable d'induire la production d'anticorps dirigés contre elle-même, ledit haptène étant susceptible de reconnaître et de se lier à une ou plusieurs molécules déjà liées, dans une première étape du traitement, à des épitopes à la surface de cellules spécifiques dans l'organisme,
- ou un peptide résultant de l'association de différents acides aminés et susceptibles de reconnaître et de se lier à des épitopes spécifiques à la surface de cellules spécifiques de l'organisme.

16. Compositions pharmaceutiques **caractérisées en ce qu'**elles contiennent une quantité efficace d'un composé selon l'une des revendications 1 à 11, ou un complexe selon l'une des revendications 13 à 15, en association avec un véhicule pharmaceutiquement acceptable.

17. Utilisation d'un complexe selon l'une des revendications 13 à 15, pour la préparation d'un médicament destiné à la radioimmunothérapie, tel que le traitement des cancers, et plus particulièrement destiné au traitement contre la prolifération des métastases.

18. Complexes selon la revendication 12, **caractérisés en ce que** les radioéléments sont des radiométaux émetteurs γ.

19. Complexes selon la revendication 18, **caractérisés en ce que** le composé est choisi parmi ceux définis dans l'une des revendications 7 à 11, et dans lesquels le groupe R comprend :
- un anticorps polyclonal ou monoclonal susceptible de reconnaître et de se lier à des épitopes spécifiques à la surface de cellules spécifiques de l'organisme,
- ou un haptène, c'est-à-dire une molécule non immunogène de faible PM capable d'induire la production d'anticorps dirigés contre elle-même, ledit haptène étant susceptible de reconnaître et de se lier à une ou plusieurs molécules déjà liées, dans une première étape de la méthode de diagnostic, à des épitopes à la surface de cellules spécifiques dans l'organisme,
- ou un peptide résultant de l'association de différents acides aminés et susceptible de reconnaître et de se lier à des épitopes spécifiques à la surface de cellules spécifiques de l'organisme.

20. Complexes selon la revendication 17 ou 18, pour la mise en oeuvre de méthodes de diagnostic telles que la radioimmunoscintigraphie.

21. Utilisation d'un composé de formule (I) tel que défini dans les revendications 1 à 11, incluant CDTPA et CTTHA, pour la fabrication d'un médicament utile en tant qu'antalgique, ou destiné au traitement des pathologies dans lesquelles des déséquilibres ioniques se produisent, ou contre la formation de calculs dans l'organisme.

22. Utilisation d'un composé de formule (I) tel que défini dans les revendications 1 à 11, incluant CDTPA et CTTHA, pour la mise en oeuvre d'un procédé de détoxification de milieux pollués, tels que des phases liquides polluées par des métaux bivalents ou trivalents, radioactifs ou non.

23. Utilisation d'un composé de formule (I) tel que défini dans les revendications 1 à 11, incluant CDTPA et CTTHA, pour la mise en oeuvre d'un procédé de purification des radionucléides, ledit composé étant lié à une phase solide.

24. Utilisation d'un composé de formule (I) tel que défini dans les revendications 1 à 11, incluant CDTPA et CTTHA, pour la mise en oeuvre d'une scintigraphie de l'os, en particulier dans le cadre du diagnostic de pathologie ostéoarticulaire, notamment dans l'équilibre de l'extension du cancer de l'os.
